# EUROPEAN PATENT APPLICATION

(11) **EP 4 199 131 A1**
(43) Date of publication of application: **21.06.2023**
(21) Application number: 21214669.0
(22) Date of filing: 15.12.2021
(51) Int. Cl.: H01L 51/54, C07C 49/92, C09K 11/06

(54) **A CE(IV) METAL COMPLEX, AN ORGANIC ELECTRONIC DEVICE COMPRISING AN ANODE LAYER, A CATHODE LAYER AND A CHARGE GENERATION LAYER, WHEREIN THE CHARGE GENERATION LAYER COMPRISES A P-TYPE CHARGE GENERATION LAYER THAT COMPRISES THE CE(IV) METAL COMPLEX AND A N-TYPE CHARGE GENERATION LAYER**

(71) Applicant: Novaled GmbH, 01099 Dresden (DE)
(72) Inventor: UVAROV, Vladimir c/o Novaled GmbH, 01099 Dresden (DE); WILLMANN, Steffen c/o Novaled GmbH, 01099 Dresden (DE); HEGGEMANN, Ulrich c/o Novaled GmbH, 01099 Dresden (DE); PINTER, Piermaria c/o Novaled GmbH, 01099 Dresden (DE)
(74) Representative: Michalski Hüttermann & Partner Patentanwälte mbB

(57) **Abstract**

The present invention is directed to an organic electronic device comprising an anode layer, a cathode layer and a charge generation layer, wherein the charge generation layer comprises a p-type charge generation layer and a n-type charge generation layer, wherein the n-type charge generation layer comprising an organic electron transport compound and a metal dopant, wherein the metal dopant is selected from a metal with an electronegativity of ≤ 1.4 eV by Pauling scale; the p-type charge generation layer comprising an organic hole transport compound and a Ce(IV) metal complex, and wherein the Ce(IV) metal complex comprises at least one anionic ligand L.

## Description

### Technical Field

The present invention relates to a Ce(IV) metal complex, an organic electronic device comprising an anode layer, a cathode layer and a charge generation layer, wherein the charge generation layer comprises a p-type charge generation layer that comprises the Ce(IV) metal complex and a n-type charge generation layer. The invention further relates to an organic electronic device comprising an anode layer, a cathode layer and a charge generation layer, wherein the charge generation layer comprises a p-type charge generation layer and a n-type charge generation layer, wherein the p-type charge generation layer comprises at least one first organic hole transport compound, and at least one Ce(IV) metal complex of a formula (I).

### Background Art

Organic electronic devices, such as organic light-emitting diodes OLEDs, which are self-emitting devices, have a wide viewing angle, excellent contrast, quick response, high brightness, excellent operating voltage characteristics, and color reproduction. A typical OLED comprises an anode, a hole transport layer HTL, an emission layer EML, an electron transport layer ETL, and a cathode, which are sequentially stacked on a substrate. In this regard, the HTL, the EML, and the ETL are thin films formed from organic compounds.

When a voltage is applied to the anode and the cathode, holes injected from the anode move to the EML, via the HTL, and electrons injected from the cathode move to the EML, via the ETL. The holes and electrons recombine in the EML to generate excitons. When the excitons drop from an excited state to a ground state, light is emitted. The injection and flow of holes and electrons should be balanced, so that an OLED having the above-described structure has excellent efficiency and/or a long lifetime.

Performance of an organic light emitting diode may be affected by characteristics of the p-type charge generation layer, and among them, may be affected by characteristics of Ce(IV) metal complexes or Ce(IV) metal complexes of formula (I), which are contained in the p-type charge generation layer.

There remains a need to improve performance of organic electronic devices comprising a charge generation layer, in particular to achieve improved operating voltage, cd/A efficiency and/or lifetime through improving the characteristics of the compounds comprised therein.

It is a further objective to provide a charge generation layer comprising compounds which can be deposited through vacuum thermal evaporation under conditions suitable for mass production.

It is a further objective to provide a charge generation layer with improved thermal properties, in particular improved thermal stability and/or rate onset temperature.

It is a further objective to provide Ce(IV) metal complexes as well as Ce(IV) metal complexes of formula (Ia) with improved physical properties, in particular improved LUMO, decomposition temperatures and/or rate onset temperatures.

### DISCLOSURE

An aspect of the present invention provides an organic electronic device comprising an anode layer, a cathode layer and a charge generation layer, wherein the charge generation layer comprises a p-type charge generation layer and a n-type charge generation layer, wherein
- the n-type charge generation layer comprising an organic electron transport compound and a metal dopant, wherein the metal dopant is selected from a metal with an electronegativity of ≤ 1.4 eV by Pauling scale;
- the p-type charge generation layer comprising an organic hole transport compound and a Ce(IV) metal complex, wherein
the Ce(IV) metal complex comprises at least one anionic ligand L, wherein
the anionic ligand comprising per ligand L at least 16 covalently bound atoms; wherein the n-type charge generation layer is arranged closer to the anode layer and the p-type charge generation layer is arranged closer to the cathode layer.

According to another embodiment the organic electronic device comprises an anode layer, a cathode layer and a charge generation layer, wherein the charge generation layer comprises a p-type charge generation layer and a n-type charge generation layer,
wherein
the n-type charge generation layer comprises an organic electron transport compound and a metal dopant; and
the p-type charge generation layer comprises:
   - an organic hole transport compound, and
   - a Ce(IV) metal complex, wherein the Ce(IV) metal complex having the formula I:

      Ce^{4⊕}(L^{⊝})₄(AL)ⁿ (I),
wherein
   - L: is an anionic ligand comprising at least 16 covalently bound atoms, wherein at least two atoms are selected from carbon atoms;
   - AL: is an ancillary ligand which coordinates to the metal M;
   - n: is an integer selected from 0 to 2, preferably 0;
wherein the n-type charge generation layer is arranged closer to the anode layer and the p-type charge generation layer is arranged closer to the cathode layer.

### Definitions

It should be noted that throughout the application and the claims the p-type charge generation layer is not a hole injection layer (HIL).

It should be noted that throughout the application and the claims any Aⁿ, Arⁿ, Rⁿ, Tⁿ etc. always refer to the same moieties, unless otherwise noted.

In the present specification, when a definition is not otherwise provided, "substituted" refers to at least one substituted with D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR³, COOR³, halogen, F or CN.

In the present specification, when a definition is not otherwise provided, a substituted aryl group with at least 6 C-ring atoms can be substituted with 1, 2, 3, 4 or 5 substituents. For example a substituted C₆ aryl group may have 1, 2, 3, 4 or 5 phenyl substituents.

However, in the present specification "aryl substituted" refers to a substitution with one or more aryl groups, which themselves may be substituted with one or more aryl and/or heteroaryl groups.

Correspondingly, in the present specification "heteroaryl substituted" refers to a substitution with one or more heteroaryl groups, which themselves may be substituted with one or more aryl and/or heteroaryl groups.

In the present specification, when a definition is not otherwise provided, a substituted heteroaryl group with at least 2 C-ring atoms can be substituted with one or more substituents. For example a substituted C₂ heteroaryl group may have 1 or 2 substituents.

In the present specification, when a definition is not otherwise provided, an "alkyl group" refers to a saturated aliphatic hydrocarbyl group. The alkyl group may be a C₁ to C₆ alkyl group. More specifically, the alkyl group may be a C₁ to C₄ alkyl group or a C₁ to C₃ alkyl group. For example, a C₁ to C₄ alkyl group includes 1 to 4 carbons in alkyl chain, and may be selected from methyl, ethyl, propyl, iso-propyl, n-butyl, iso-butyl, sec-butyl, and tert-butyl.

Specific examples of the alkyl group may be a methyl group, an ethyl group, a propyl group, an iso-propyl group, a butyl group, an iso-butyl group, a sec-butyl group, a tert-butyl group, a pentyl group, a branched pentyl group, a hexyl group.

The term "cycloalkyl" refers to saturated hydrocarbyl groups derived from a cycloalkane by formal abstraction of one hydrogen atom from a ring atom comprised in the corresponding cycloalkane. Examples of the cycloalkyl group may be a cyclopropyl group, a cyclobutyl group, a cyclopentyl group, a cyclohexyl group, a methyl cyclohexyl group, an adamantly group and the like.

The term "hetero" is understood the way that at least one carbon atom, in a structure which may be formed by covalently bound carbon atoms, is replaced by another polyvalent atom. Preferably, the heteroatoms are selected from N, P, O, S, F; more preferably from N, O, F.

In the present specification, "aryl group" and "aromatic rings" refers to a hydrocarbyl group which can be created by formal abstraction of one hydrogen atom from an aromatic ring in the corresponding aromatic hydrocarbon. Aromatic hydrocarbon refers to a hydrocarbon which contains at least one aromatic ring or aromatic ring system. Aromatic ring or aromatic ring system refers to a planar ring or ring system of covalently bound carbon atoms, wherein the planar ring or ring system comprises a conjugated system of delocalized electrons fulfilling Hückel's rule. Examples of aryl groups include monocyclic groups like phenyl or tolyl, polycyclic groups which comprise more aromatic rings linked by single bonds, like biphenyl, and polycyclic groups comprising fused rings, like naphthyl or fluorenyl.

Analogously, under "heteroaryl" and "heteroaromatic", it is especially where suitable understood a group derived by formal abstraction of one ring hydrogen from a heterocyclic aromatic ring in a compound comprising at least one such ring.

The term "non-heterocycle" is understood to mean a ring or ring-system comprising no hetero-atom as a ring member.

The term "heterocycle" is understood to mean that the heterocycle comprises at least one ring comprising one or more hetero-atoms. A heterocycle comprising more than one ring means that all rings comprising a hetero-atom or at least one ring comprising a hetero atom and at least one ring comprising C-atoms only and no hetero atom.

Under heterocycloalkyl, it is especially where suitable understood a group derived by formal abstraction of one ring hydrogen from a saturated cycloalkyl ring in a compound comprising at least one such ring.

The term "fused aryl rings" or "condensed aryl rings" is understood the way that two aryl rings are considered fused or condensed when they share at least two common sp²-hybridized carbon atoms.

The term "fused ring system" is understood to mean a ring system wherein two or more rings share at least two atoms.

The term "5-, 6- or 7-member ring" is understood to mean a ring comprising 5, 6 or 7 atoms. The atoms may be selected from C and one or more hetero-atoms.

In the present specification, the single bond refers to a direct bond.

In the present specification, when a definition is not otherwise provided, "substituted" refers to one substituted with a H, deuterium, C₁ to C₁₂ alkyl, unsubstituted C₆ to C₁₈ aryl, and unsubstituted C₃ to C₁₈ heteroaryl.

In the present specification, when a substituent is not named, the substituent can be a H.

In the context of the present invention, "different" means that the compounds do not have an identical chemical structure.

The term "cyano moiety" refers to a CN substituent.

In the present specification, the single bond refers to a direct bond.

The term "free of', "does not contain", "does not comprise" does not exclude impurities which may be present in the compounds prior to deposition. Impurities have no technical effect with respect to the object achieved by the present invention.

The term "contacting sandwiched" refers to an arrangement of three layers whereby the layer in the middle is in direct contact with the two adjacent layers.

The terms "light-absorbing layer" and "light absorption layer" are used synonymously.

The terms "light-emitting layer", "light emission layer" and "emission layer" are used synonymously.

The terms "OLED", "organic light-emitting diode" and "organic light-emitting device" are used synonymously.

The terms anode, anode layer and anode electrode are used synonymously.

The terms cathode, cathode layer and cathode electrode are used synonymously.

The term "n-type charge generation layer" may also be named n-type CGL or n-CGL or electron generation layer. These terms are used synonymously.

The term "n-type charge generation layer" is understood to mean a layer which improves electron generation and transport layer into further layers in the organic electronic device.

The term "p-type charge generation layer" may also be named p-type CGL or p-CGL or hole generation layer. These terms are used synonymously.

The term "p-type charge generation layer" is understood to mean a layer which improves hole generation and transport into further layers in the organic electronic device.

The term "charge generation connecting layer" is may also be named c-CGL or interlayer or buffer layer. These terms are used synonymously.

The term "charge generation connecting layer" is understood to mean a layer which enables charge generation and transport between the n-type CGL and the p-type CGL.

The term "hole injection layer" is understood to mean a layer which enables hole injection from the anode layer into further layers in the organic electronic device.

The term "hole transport layer" is understood to mean a layer which transports holes between the anode layer and the cathode layer.

The term "top emission device" is understood to mean an organic electronic device wherein the light is emitted through the cathode layer.

The term "bottom emission device" is understood to mean an organic electronic device wherein the light is emitted through the substrate.

In the specification, hole characteristics refer to an ability to donate an electron to form a hole when an electric field is applied and that a hole formed in the anode may be easily injected into the emission layer and transported in the emission layer due to conductive characteristics according to a highest occupied molecular orbital (HOMO) level.

In addition, electron characteristics refer to an ability to accept an electron when an electric field is applied and that electrons formed in the cathode may be easily injected into the emission layer and transported in the emission layer due to conductive characteristics according to a lowest unoccupied molecular orbital (LUMO) energy.

The term "HOMO energy" is understood to mean the highest occupied molecular orbital and is determined in eV (electron volt).

The term "HOMO energy further away from vacuum level" is understood to mean that the absolute value of the HOMO energy is higher than the absolute value of the HOMO energy of the reference compound. For example, the term "further away from vacuum level than the HOMO energy of N2,N2,N2',N2',N7,N7,N7',N7'-octakis(4-methoxyphenyl)-9,9'-spirobi[fluorene]-2,2',7,7'-tetraamine is understood to mean that the absolute value of the HOMO energy of the organic hole transport compound of the p-type charge generation layer is higher than the HOMO energy of N2,N2,N2',N2', N7,N7, N7',N7'-octakis(4-methoxyphenyl)-9,9'-spirobi[fluorene]-2,2',7,7'-tetraamine.

The term "absolute value" is understood to mean the value without the "-" symbol.

The electronegativity according to Pauling, also named Pauling scale, is measured in eV (electron volt). Tabulated values of the electronegativity according to Pauling can be found for example in "John Emsley, The Elements, 3rd edition. Oxford: Clarendon Press, 1998". Further tabulated values can be found for example at
https://en.wikipedia.org/wiki/Electronegativities_of_the_elements_(data_page).

### Advantageous Effects

Surprisingly, it was found that the organic electronic device according to the invention solves the problem underlying the present invention by enabling organic electronic devices, such as organic light-emitting diodes, in various aspects superior over the organic electronic devices known in the art, in particular with respect to operating voltage, cd/A efficiency and/or lifetime.

Additionally, it was found that the problem underlying the present invention can be solved by providing compounds which may be suitable for deposition through vacuum thermal evaporation under conditions suitable for mass production. In particular, the thermal stability and/or rate onset temperature of the Ce(IV) metal complex and/or the organic hole transport compound of the present invention may be in a range suitable for mass production.

Additionally, the LUMO of the Ce(IV) metal complex or Ce(IV) metal complex of formula (I) or (Ia) may be particularly suitable for efficient hole generation in the p-type charge generation layer.

### Embodiment of the organic electronic device

According to one embodiment of the organic electronic device, wherein the LUMO energy of the Ce(IV) metal complex or Ce(IV) metal complex of formula (I) is selected ≤ -4.6 eV and ≥ 6 eV, preferably ≤ -4.65 eV and ≥ 6 eV, also preferred ≤ -4.7 eV and ≥ 6 eV, further preferred ≤ -4.8 eV and ≥ 6 eV; wherein the LUMO energy is calculated as single point energy calculation with the program package TURBOMOLE V6.5 (TURBOMOLE GmbH, Litzenhardtstrasse 19, 76135 Karlsruhe, Germany) from the optimized geometry by applying the hybrid functional B3LYP with a Def-TZVP basis set in the gas phase and inclusion of dispersion forces as D3BJ. Geometry is optimized by applying the hybrid functional B3LYP with a Def-SVP basis set in the gas phase and inclusion of dispersion forces as D3BJ. If more than one conformation is viable, the conformation with the lowest total energy is selected.

According to one embodiment the organic electronic device comprises an anode layer, a cathode layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from a metal with an electronegativity of ≤ 1.4 eV by Pauling scale;
- the p-type charge generation layer comprising an organic hole transport compound and a Ce(IV) metal complex, wherein
the Ce(IV) metal complex comprises at least one anionic ligand L, wherein
the anionic ligand comprising per ligand L ≥ 16 covalently bound atoms and ≤ 80 covalently bound atoms;
wherein the n-type charge generation layer is arranged closer to the anode layer and the p-type charge generation layer is arranged closer to the cathode layer.

According to another embodiment the organic electronic device comprises an anode layer, a cathode layer and a charge generation layer, wherein the charge generation layer comprises a p-type charge generation layer and a n-type charge generation layer,
wherein
the n-type charge generation layer comprises an organic electron transport compound and a metal dopant, wherein the metal dopant is selected from a metal with an electronegativity of ≤ 1.4 eV by Pauling scale; and
the p-type charge generation layer comprises:
   - an organic hole transport compound, and
   - a Ce(IV) metal complex, wherein the Ce(IV) metal complex having the formula I:

      Ce^{4⊕}(L^{⊝})₄(AL)ₙ (I),
wherein
   - L: is an anionic ligand comprising at least 16 covalently bound atoms, wherein at least two atoms are selected from carbon atoms;
   - AL: is an ancillary ligand which coordinates to the metal M;
   - n: is an integer selected from 0 to 2, preferably 0;
wherein the n-type charge generation layer is arranged closer to the anode layer and the p-type charge generation layer is arranged closer to the cathode layer.

According to one embodiment, "n" is an integer selected from 0, 1 or 2, preferably 2, further preferred 1 and in addition preferred 0.

According to another embodiment the organic electronic device comprises an anode layer, a cathode layer and a charge generation layer, wherein the charge generation layer comprises a p-type charge generation layer and a n-type charge generation layer,
wherein the n-type charge generation layer comprises an organic electron transport compound and a metal dopant, wherein the metal dopant is selected from a metal comprising Li, Na, K, Rb, Cs, Mg, Ca, Sr, Ba, Sm, Eu or Yb, more preferred from Li, Na, K, Cs, Mg, Ca, Ba, Sm, Eu or Yb, even more preferred from Li, Mg or Yb; and
the p-type charge generation layer comprises:
   - an organic hole transport compound, and
   - a Ce(IV) metal complex, wherein the Ce(IV) metal complex having the formula I:

      Ce^{4⊕}(L^{⊝})₄(AL)ₙ (I),
wherein
   - L: is an anionic ligand comprising at least 16 covalently bound atoms, wherein at least two atoms are selected from carbon atoms;
   - AL: is an ancillary ligand which coordinates to the metal M;
   - n: is an integer selected from 0 to 2, preferably 0;
wherein the n-type charge generation layer is arranged closer to the anode layer and the p-type charge generation layer is arranged closer to the cathode layer.

According to one embodiment the organic electronic device comprises an anode layer, a cathode layer and a charge generation layer, wherein the charge generation layer comprises a p-type charge generation layer and a n-type charge generation layer:
i) wherein
   - the n-type charge generation layer comprising an organic electron transport compound and a metal dopant, wherein the metal dopant is selected from a metal with an electronegativity of ≤ 1.4 eV by Pauling scale;
   - the p-type charge generation layer comprising an organic hole transport compound and a Ce(IV) metal complex, wherein
      the Ce(IV) metal complex comprises at least one anionic ligand L, wherein the anionic ligand comprising per ligand L ≥ 16 covalently bound atoms and ≤ 80 covalently bound atoms; or
ii) wherein
   - the p-type charge generation layer comprises:
   - an organic hole transport compound, and
   - a Ce(IV) metal complex, wherein the Ce(IV) metal complex having the formula I:

      Ce^{4⊕}(L^{⊝})₄(AL)ₙ (I),
wherein
- L: is an anionic ligand comprising ≥ 16 covalently bound atoms and ≤ 80 covalently bound atoms, wherein at least two atoms are selected from carbon atoms;
- AL: is an ancillary ligand which coordinates to the metal M;
- n: is an integer selected from 0 to 2, preferably 0;
wherein the n-type charge generation layer is arranged closer to the anode layer and the p-type charge generation layer is arranged closer to the cathode layer.

According to one embodiment the organic electronic device comprises an anode layer, a cathode layer and a charge generation layer, wherein the charge generation layer comprises a p-type charge generation layer and a n-type charge generation layer:
i) wherein
   - the n-type charge generation layer comprising an organic electron transport compound and a metal dopant, wherein the metal dopant is selected from a metal with an electronegativity of ≤ 1.4 eV by Pauling scale;
   - the p-type charge generation layer comprising an organic hole transport compound and a Ce(IV) metal complex, wherein
      the Ce(IV) metal complex comprises at least one anionic ligand L, wherein
      the anionic ligand comprising per ligand L ≥ 16 covalently bound atoms and ≤ 80 covalently bound atoms; or
ii) wherein
   - the n-type charge generation layer comprising an organic electron transport compound and a metal dopant, wherein the metal dopant is selected from a metal with an electronegativity of ≤ 1.4 eV by Pauling scale;
   - the p-type charge generation layer comprises:
   - an organic hole transport compound, and
   - a Ce(IV) metal complex, wherein the Ce(IV) metal complex having the formula I:

      Ce^{4⊕}(L^{⊝})₄(AL)ₙ (I),
   wherein
   - L: is an anionic ligand comprising ≥ 16 covalently bound atoms and ≤ 80 covalently bound atoms, wherein at least two atoms are selected from carbon atoms;
   - AL: is an ancillary ligand which coordinates to the metal M;
   - n: is an integer selected from 0 to 2, preferably 0;
   wherein the n-type charge generation layer is arranged closer to the anode layer and the p-type charge generation layer is arranged closer to the cathode layer.

According to one embodiment the organic electronic device comprises an anode layer, a cathode layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from a metal with an electronegativity of ≤ 1.4 eV by Pauling scale;
- the p-type charge generation layer comprising an organic hole transport compound and a Ce(IV) metal complex, wherein
   the Ce(IV) metal complex comprises at least one anionic ligand L, wherein the anionic ligand comprising per ligand L ≥ 16 covalently bound atoms and ≤ 80 covalently bound atoms, wherein ≥ 8 atoms and ≤ 24 are selected from carbon atoms and ≥ 4 atoms and ≤ 24 are selected from hetero atoms selected from the group comprising N, O and F, preferably ≥ 8 atoms and ≤ 24 are selected from carbon atoms and ≥ 4 atoms and ≤ 24 are selected from hetero atoms selected from the group comprising N, O and F, wherein the number of O atoms is 2 and the number of N atoms is 0 to 4, preferably the number of N atoms is 0, 1 or 2;
wherein the n-type charge generation layer is arranged closer to the anode layer and the p-type charge generation layer is arranged closer to the cathode layer.

According to one embodiment the organic electronic device comprises an anode layer, a cathode layer and a charge generation layer, wherein the charge generation layer comprises a p-type charge generation layer and a n-type charge generation layer:
i) wherein
   - the n-type charge generation layer comprising an organic electron transport compound and a metal dopant, wherein the metal dopant is selected from a metal with an electronegativity of ≤ 1.4 eV by Pauling scale;
   - the p-type charge generation layer comprising an organic hole transport compound and a Ce(IV) metal complex, wherein
      the Ce(IV) metal complex comprises at least one anionic ligand L, wherein the anionic ligand comprising per ligand L ≥ 16 covalently bound atoms and ≤ 80 covalently bound atoms, wherein ≥ 8 atoms and ≤ 24 are selected from carbon atoms and ≥ 4 atoms and ≤ 24 are selected from hetero atoms selected from the group comprising N, O and F, preferably ≥ 8 atoms and ≤ 24 are selected from carbon atoms and ≥ 4 atoms and ≤ 24 are selected from hetero atoms selected from the group comprising N, O and F, wherein the number of O atoms is 2 and the number of N atoms is 0 to 4, preferably the number of N atoms is 0, 1 or 2.; or
ii) wherein
   - the p-type charge generation layer comprises:
   - an organic hole transport compound, and
   - a Ce(IV) metal complex, wherein the Ce(IV) metal complex having the formula I:

      Ce^{4⊕}(L^{⊝})₄(AL)ₙ (I),
wherein
- L: is an anionic ligand comprising ≥ 16 covalently bound atoms and ≤ 80 covalently bound atoms, wherein ≥ 8 atoms and ≤ 24 are selected from carbon atoms and ≥ 4 atoms and ≤ 24 are selected from hetero atoms selected from the group comprising N, O and F, preferably ≥ 8 atoms and ≤ 24 are selected from carbon atoms and ≥ 4 atoms and ≤ 24 are selected from hetero atoms selected from the group comprising N, O and F, wherein the number of O atoms is 2 and the number of N atoms is 0 to 4, preferably the number of N atoms is 0, 1 or 2;
- AL: is an ancillary ligand which coordinates to the metal M;
- n: is an integer selected from 0 to 2, preferably 0;
wherein the n-type charge generation layer is arranged closer to the anode layer and the p-type charge generation layer is arranged closer to the cathode layer.

According to one embodiment the organic electronic device comprises an anode layer, a cathode layer and a charge generation layer, wherein the charge generation layer comprises a p-type charge generation layer and a n-type charge generation layer:
i) wherein
   - the n-type charge generation layer comprising an organic electron transport compound and a metal dopant, wherein the metal dopant is selected from a metal with an electronegativity of ≤ 1.4 eV by Pauling scale;
   - the p-type charge generation layer comprising an organic hole transport compound and a Ce(IV) metal complex, wherein
      the Ce(IV) metal complex comprises at least one anionic ligand L, wherein the anionic ligand comprising per ligand L ≥ 16 covalently bound atoms and ≤ 80 covalently bound atoms, wherein ≥ 8 atoms and ≤ 24 are selected from carbon atoms and ≥ 4 atoms and ≤ 24 are selected from hetero atoms selected from the group comprising N, O and F, preferably ≥ 8 atoms and ≤ 24 are selected from carbon atoms and ≥ 4 atoms and ≤ 24 are selected from hetero atoms selected from the group comprising N, O and F, wherein the number of O atoms is 2 and the number of N atoms is 0 to 4, preferably the number of N atoms is 0, 1 or 2; or
ii) wherein
   - the n-type charge generation layer comprising an organic electron transport compound and a metal dopant, wherein the metal dopant is selected from a metal with an electronegativity of ≤ 1.4 eV by Pauling scale;
   - the p-type charge generation layer comprises:
   - an organic hole transport compound, and
   - a Ce(IV) metal complex, wherein the Ce(IV) metal complex having the formula I:

      Ce^{4⊕}(L^{⊝})₄(AL)ₙ (I),
wherein
- L: is an anionic ligand comprising ≥ 16 covalently bound atoms and ≤ 80 covalently bound atoms, wherein ≥ 8 atoms and ≤ 24 are selected from carbon atoms and ≥ 4 atoms and ≤ 24 are selected from hetero atoms selected from the group comprising N, O and F, preferably ≥ 8 atoms and ≤ 24 are selected from carbon atoms and ≥ 4 atoms and ≤ 24 are selected from hetero atoms selected from the group comprising N, O and F, wherein the number of O atoms is 2 and the number of N atoms is 0 to 4, preferably the number of N atoms is 0, 1 or 2;
- AL: is an ancillary ligand which coordinates to the metal M;
- n: is an integer selected from 0 to 2, preferably 0;
wherein the n-type charge generation layer is arranged closer to the anode layer and the p-type charge generation layer is arranged closer to the cathode layer.

### Ancillary ligand AL

Ancillary ligands are defined as those ligands that provide the appropriate steric and
electronic environment around the central element but remain innocent in any transformation that the compound undergoes. In contrast, reactive ligands are those groups that undergo changes.

According to one embodiment of the organic electronic device, wherein AL is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (II); 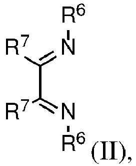 wherein
- R⁶ and R⁷: are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

### Ligand L of formula (I)

According to one embodiment of the organic electronic device, wherein the ligand L of formula I comprising per ligand L at least 16 covalently bound atoms; or
the ligand L of formula I comprising:
- at least three carbon atoms, alternatively at least four carbon atoms, and/or
- at least two oxygen atoms or one oxygen and one nitrogen atom, two to four oxygen atoms, two to four oxygen atoms and zero to two nitrogen atoms, and/or
- at least one or more groups selected from halogen, F, CN, substituted or unsubstituted C₁ to C₆ alkyl, substituted or unsubstituted C₁ to C₆ alkoxy, alternatively two or more groups selected from halogen, F, CN, substituted or unsubstituted C₁ to C₆ alkyl, substituted or unsubstituted C₁ to C₆ alkoxy, at least one or more groups selected from halogen, F, CN, substituted C₁ to C₆ alkyl, substituted C₁ to C₆ alkoxy, alternatively two or more groups selected from halogen, F, CN, perfluorinated C₁ to C₆ alkyl, perfluorinated C₁ to C₆ alkoxy, one or more groups selected from substituted or unsubstituted C₁ to C₆ alkyl, substituted or unsubstituted C₆ to C₁₂ aryl, and/or substituted or unsubstituted C₃ to C₁₂ heteroaryl,
wherein the substituents are selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR³, COOR³, halogen, F or CN; wherein R³ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to one embodiment of the organic electronic device, wherein the ligand L of formula (I) may be selected from G1 to G60:

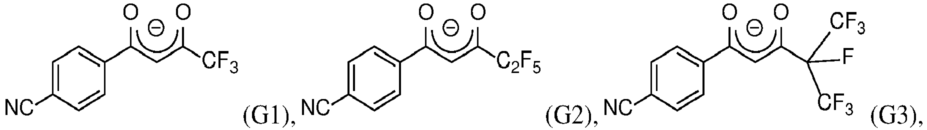

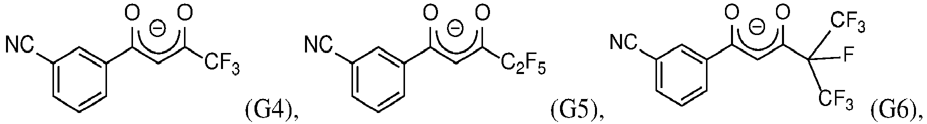

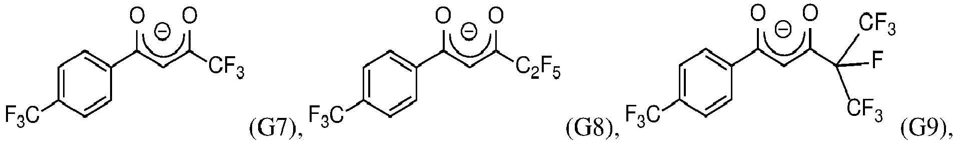

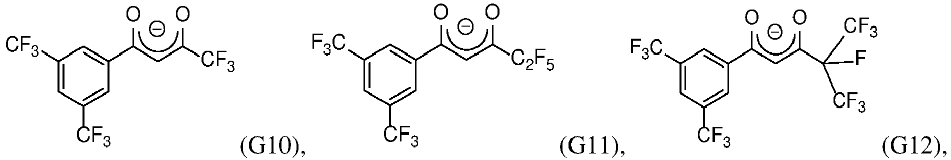

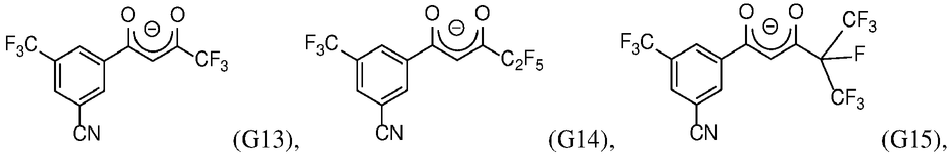

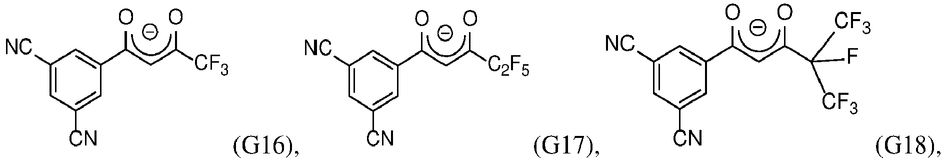

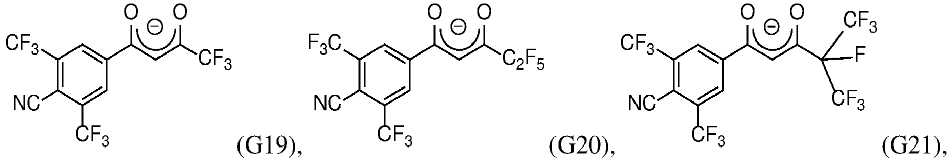

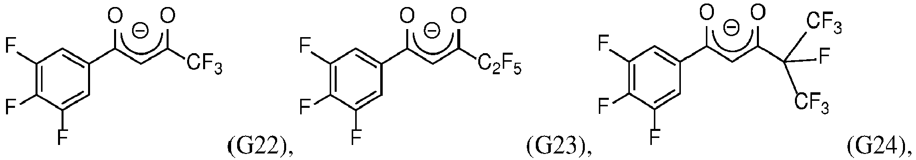

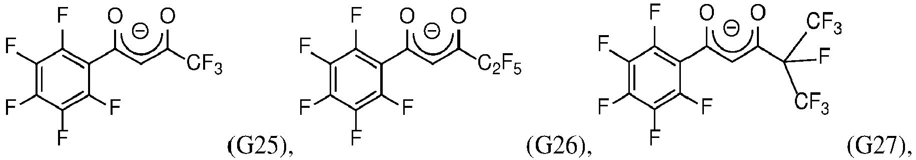

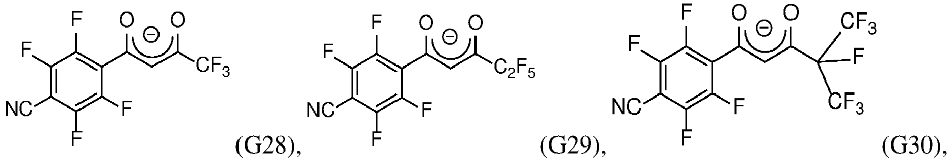

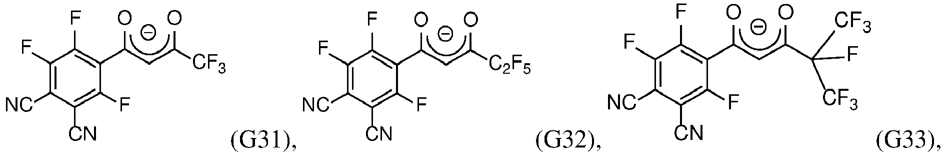

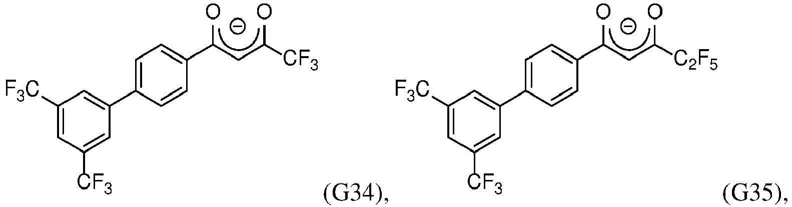

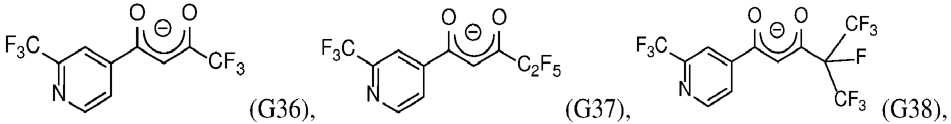

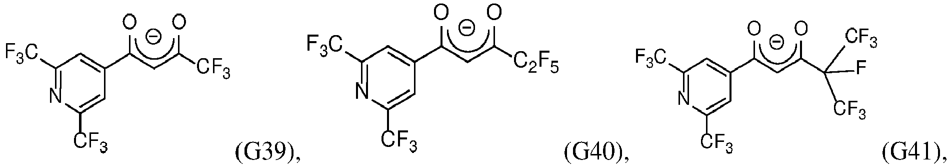

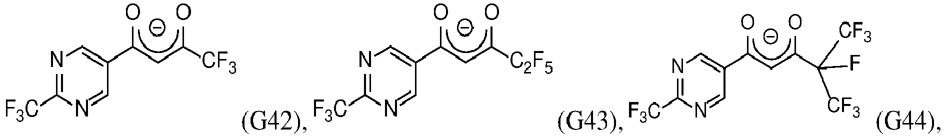

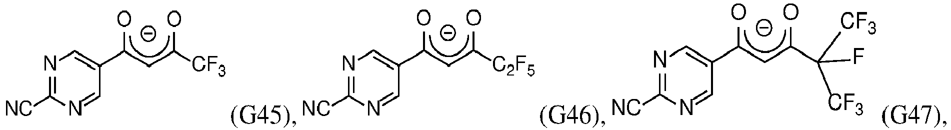

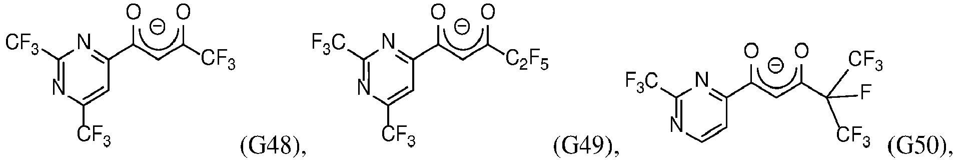

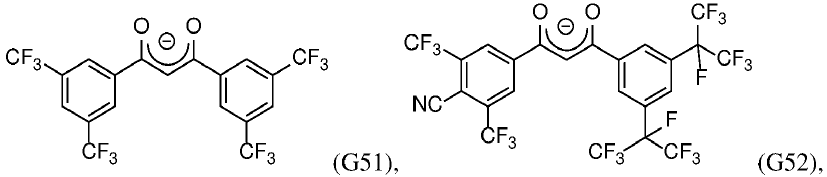

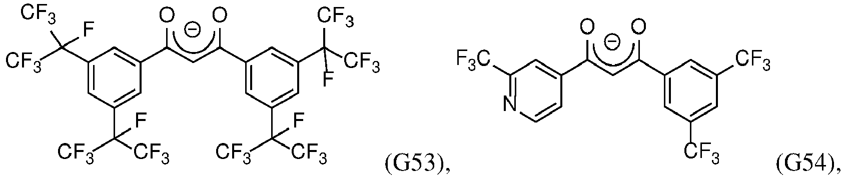

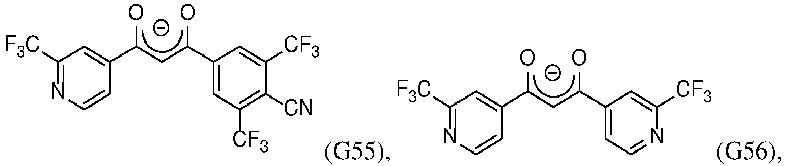

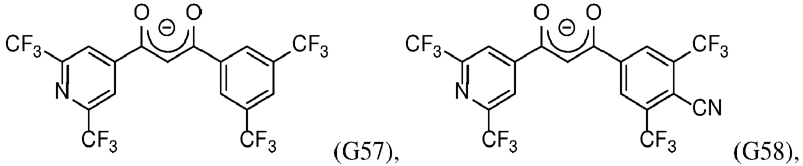

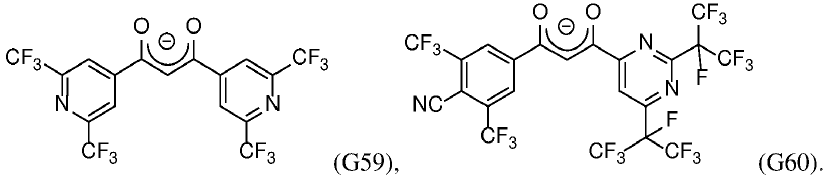

According to one embodiment of the organic electronic device, L of formula (I) is selected from (G1) to (G50) and (G52) to (G60), preferably from (G1) to (G50).

### Ce(IV) metal complex and Ce(IV) metal complex of formula (I)

The Ce(IV) metal complex and Ce(IV) metal complex of formula (I) are non-emissive. In the context of the present specification the term "essentially non-emissive" or "non-emissive" means that the contribution of the metal complex according to formula (I) to the visible emission spectrum from an organic electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

According to one embodiment of the organic electronic device, the valency of the Ce metal of the Ce(IV) metal complex and Ce(IV) metal complex of formula (I) is 4.

According to one embodiment of the organic electronic device, wherein the Ce(IV) metal complex and the Ce(IV) metal complex of formula (I) may have a molecular weight Mw of ≥ 1000 g/mol and ≤ 1600 g/mol, further preferred a molecular weight Mw of ≥ 1000 g/mol and ≤ 1500 g/mol.

### Ce(IV) metal complex of formula (Ia)

According to one embodiment of the organic electronic device, wherein the Ce(IV) metal complex has the formula (Ia): 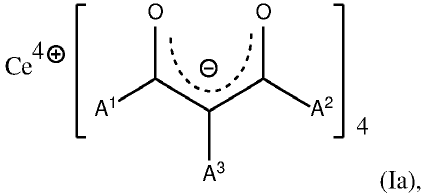 wherein
- A¹ and A²: are independently selected from substituted or unsubstituted C₁ to C₁₂ alkyl, substituted or unsubstituted C₆ to C₁₂ aryl, substituted or unsubstituted C₃ to C₁₂ heteroaryl, preferably substituted C₁ to C₁₂ alkyl, substituted C₆ to C₁₂ aryl, substituted C₃ to C₁₂ heteroaryl;
- A³: is selected from H or D, preferably H;
wherein
the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN,
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C6 cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to one embodiment, wherein A¹ and A² may be selected different.

According to one embodiment of the organic electronic device, wherein the LUMO energy of the Ce(IV) metal complex of formula (Ia) is selected ≤ -4.6 eV and ≥ 6 eV, preferably ≤ -4.65 eV and ≥ 6 eV, also preferred ≤ -4.7 eV and ≥ 6 eV, further preferred ≤ -4.8 eV and ≥ 6 eV; wherein the LUMO energy is calculated as single point energy calculation with the program package TURBOMOLE V6.5 (TURBOMOLE GmbH, Litzenhardtstrasse 19, 76135 Karlsruhe, Germany) from the optimized geometry by applying the hybrid functional B3LYP with a Def-TZVP basis set in the gas phase and inclusion of dispersion forces as D3BJ. Geometry is optimized by applying the hybrid functional B3LYP with a Def-SVP basis set in the gas phase and inclusion of dispersion forces as D3BJ. If more than one conformation is viable, the conformation with the lowest total energy is selected

According to one embodiment of the organic electronic device, wherein
- the substituents on A¹ and A² are independently selected from halogen, preferably F, C₁ to C₃ perhalogenated, perfluorinated alkyl or perfluorinated alkoxy, preferably perfluorinated C₁ to C₃ alkyl or perfluorinated C₁ to C₃ alkoxy, or -(O)₁-CₘH₂ₘ-CₙHaloₙ₂ₙ₊₁ with 1= 0 or 1, preferably 0, m = 1 or 2, preferably 1 and n = 1 to 3, preferably n =1 or 2 and Halo = halogen, preferably F; and/or
- at least one of A¹ and A² is substituted alkyl and the substituents of the alkyl are fluorine with the number n_{F} (of fluorine substituents) and n_{H} (of hydrogens) follow the equation: n_{F} > n_{H} + 2; and/or
- at least one of A¹ and A² is selected from perfluorinated C₁ to C₆ alkyl, phenyl substituted with F or CF₃; and/or
- at least one of A¹ and A² is selected from perfluorinated alkyl or aryl.

According to one embodiment of the organic electronic device, wherein the substituents on A¹ and A² are independently selected from halogen, preferably F, C₁ to C₃ perhalogenated, perfluorinated alkyl or perfluorinated alkoxy, preferably perfluorinated C₁ to C₃ alkyl or perfluorinated C₁ to C₃ alkoxy, or -(O)ₗ-CₘH₂ₘ-CnHaloₙ₂ₙ₊₁ with 1= 0 or 1, preferably 0, m = 1 or 2, preferably 1 and n = 1 to 3, preferably n =1 or 2 and Halo = halogen, preferably F.

According to one embodiment of the organic electronic device, wherein at least one of A¹ and A² is substituted alkyl and the substituents of the alkyl are fluorine with the number n_{F} (of fluorine substituents) and n_{H} (of hydrogens) follow the equation: n_{F} > n_{H} + 2.

According to one embodiment of the organic electronic device, wherein at least one of A¹ and A² is selected from perfluorinated C₁ to C₆ alkyl, phenyl substituted with F or CF.

According to one embodiment of the organic electronic device, wherein at least one of A¹ and A² is selected from perfluorinated alkyl or perfluorinated aryl, preferably perfluorinated C₁ to C₃ alkyl.

According to one embodiment of the organic electronic device, wherein at least one of A¹ and A² comprises a substituent, wherein at least one of the substituents of A¹ and A² are independently selected from C₃ to C₉ heteroaryl, C₁ to C₆ alkoxy, C₃ to C₆ branched alkoxy, C₃ to C6 cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; preferably at least one of A¹ and A² comprises at least two substituents, wherein the substituents on A¹ and A² are independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; and further preferred A¹ and A² comprise at least one substituent independently selected from halogen, F, CF₃, C₂F₅, C₃F₇, C₄F₉, OCF₃, OC₂F₅, or CN; and in addition preferred A¹ and A² may comprise at least two substituents independently selected from halogen, F, CF₃, C₂F₅, C₃F₇, C₄F₉, OCF₃, OC₂F₅ or CN;
wherein R¹ is selected from C₆ aryl, C₃ to C₉ heteroaryl, C₁ to C₆ alkyl, C₁ to C₆ alkoxy, C₃ to C6 branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to one embodiment of the organic electronic device, wherein the sum of A¹ and A² comprise ≥ 3 carbon atoms and ≤ 30 carbon atoms.

Preferably the sum of A¹ and A² comprise ≥ 5 carbon atoms and ≤ 28 carbon atoms, further preferred the sum of A¹ and A² comprise ≥ 7 carbon atoms and ≤ 26 carbon atoms, in addition preferred the sum of A¹ and A² comprise ≥ 8 carbon atoms and ≤ 24 carbon atoms, also preferred the sum of A¹ and A² comprise ≥ 9 carbon atoms and ≤ 22 carbon atoms, or the sum of A¹ and A² comprise ≥ 10 carbon atoms and ≤ 20 carbon atoms, or the sum of A¹ and A² comprise ≥ 11 carbon atoms and ≤ 18 carbon atoms, or the sum of A¹ and A² comprise ≥ 12 carbon atoms and ≤ 16 carbon atoms, the sum of A¹ and A² comprise ≥ 13 carbon atoms and ≤ 15 carbon atoms.

According to one embodiment of the present invention, A² is aryl or heteroaryl, whereby the substituents of the aryl and/or heteroaryl moiety are selected from hydrogen, halogen, F, CN or trifluoro methyl.

According to one embodiment of the present invention A² is phenyl or six-membered heteroaryl, which is substituted with 1 to 5 F atoms.

According to one embodiment of the present invention, A¹ is substituted or unsubstituted C₁ to C₆ alkyl or substituted phenyl and A² is substituted C₃ to C₆ alkyl; alternatively, A¹ is substituted or unsubstituted C₁ to C₄ alkyl or substituted phenyl and A² is substituted C₃ to C₄ alkyl or substituted phenyl.

According to one embodiment, wherein A³ is selected from H or D, preferably H and A¹ and A² may be independently selected from substituted or unsubstituted C₁ to C₁₂ alkyl, substituted or unsubstituted C₆ to C₁₂ aryl, substituted or unsubstituted C₃ to C₁₂ heteroaryl; wherein at least one of A¹ and A² may comprises a substituent, wherein at least one of the substituents of A¹ and A² may be independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; preferably at least one of A¹ and A² may comprises at least two substituents, wherein the substituents on A¹ and A² may be independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; and further preferred A¹ and A² comprise at least one substituent independently selected from halogen, F, CF₃, C₂F₅, C₃F₇, C₄F₉, OCF₃, OC₂F₅, or CN; and in addition preferred A¹ and A² may comprise at least two substituents independently selected from halogen, F, CF₃, C₂F₅, C₃F₇, C₄F₉, OCF₃, OC₂F₅ or CN.

According to one embodiment, wherein the Ce(IV) metal complex of formula (I) is free of alkoxy, COR¹ and/or COOR¹ groups, wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

According to one embodiment, wherein A¹ and/or A² are selected from the following Formulas D1 to D68: 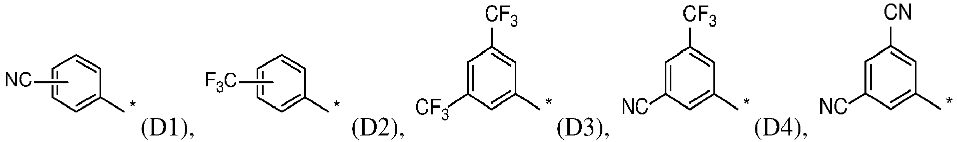 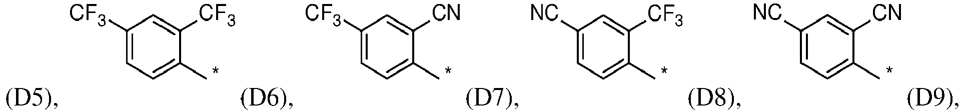 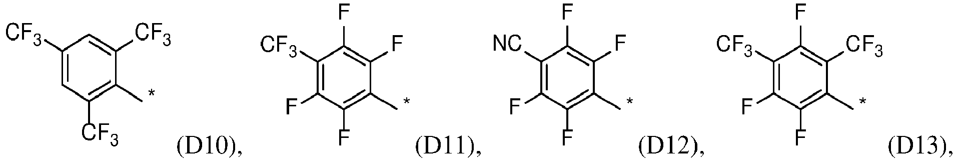 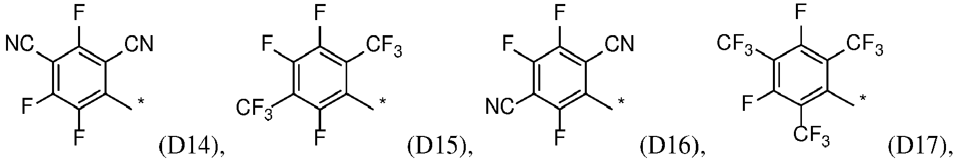 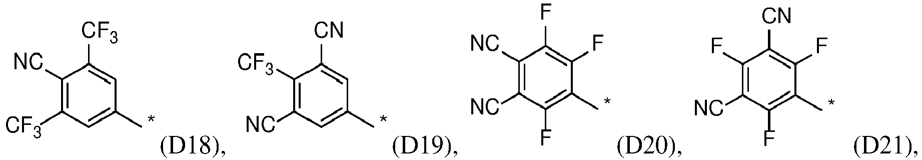 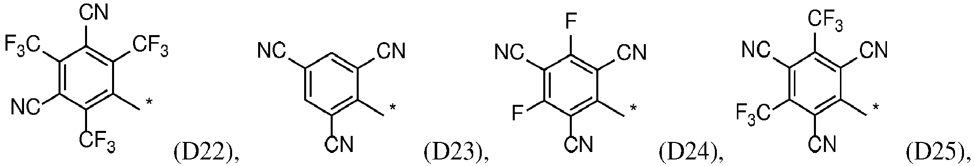 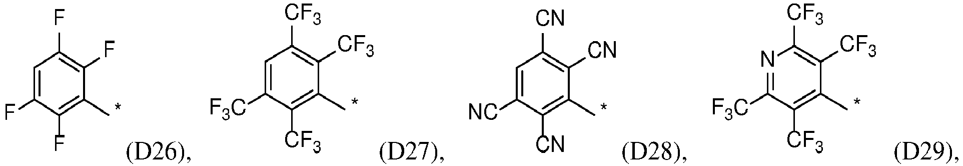 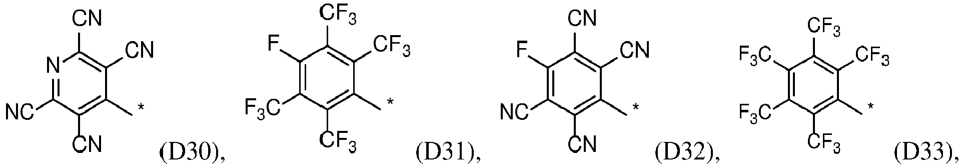 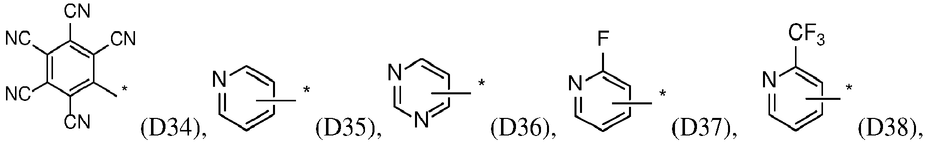 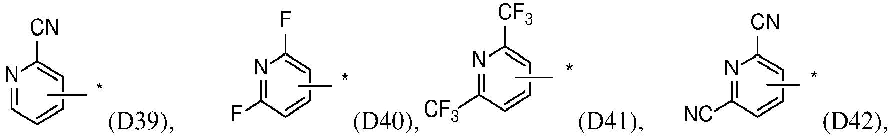 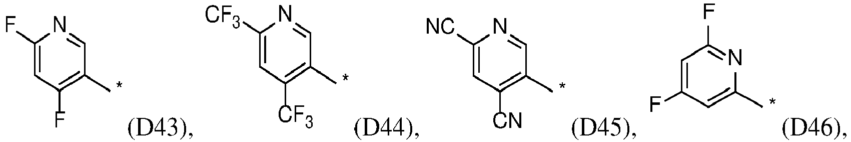 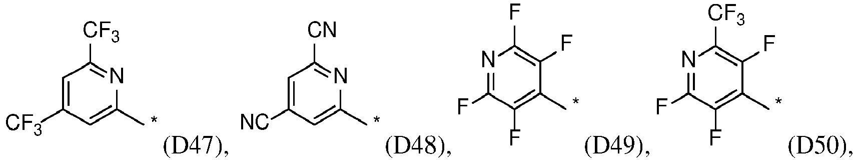 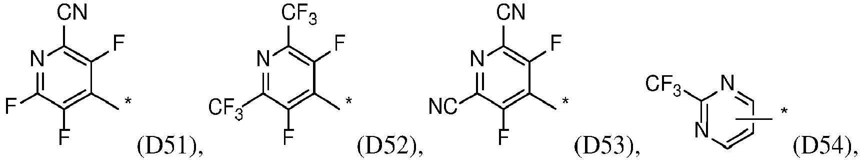 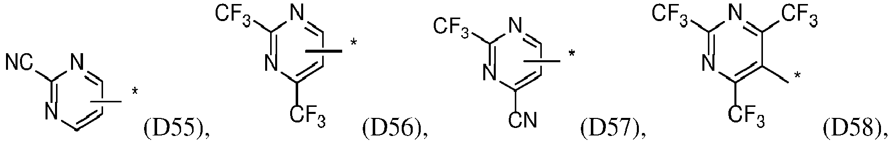 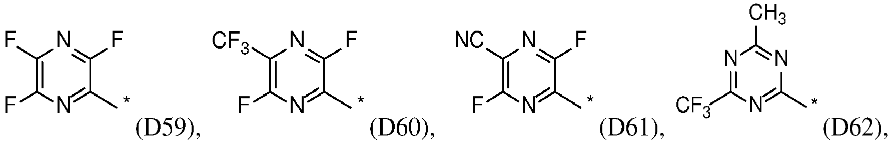 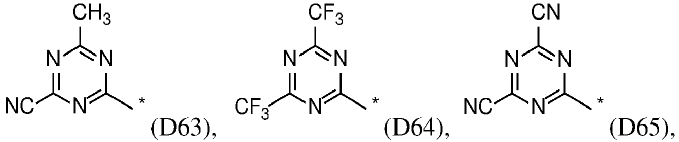 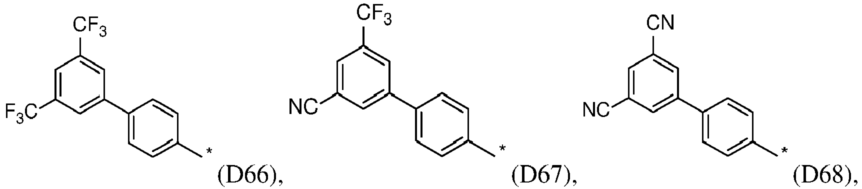 wherein the "^{∗}" denotes the binding position.

According to one embodiment, wherein the Ce(IV) metal complex may be selected from the above formula (Ia), wherein A¹ is selected from formula D1 to D68 and A² is selected from substituted or unsubstituted C₁ to C₁₂ alkyl, preferably substituted C₁ to C₁₂ alkyl.

According to one embodiment of the organic electronic device, wherein the Ce(IV) metal complex has the formula (Ia): 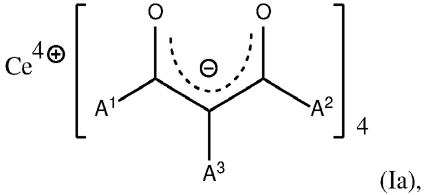 wherein
- A¹: is selected from formulae D1 and D4: 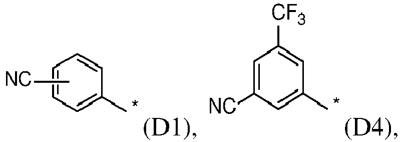 wherein the "^{∗}" denotes the binding position;
- A²: is selected from substituted or unsubstituted C₁ to C₁₂ alkyl, wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
- A³: is selected from H or D, preferably H; or
wherein
- A¹: is selected from formulae D39, D42 and D56: 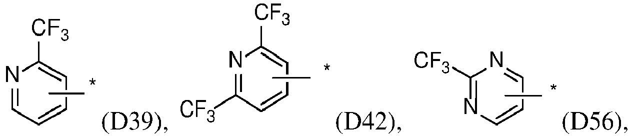
wherein the "^{∗}" denotes the binding position;
- A²: is selected from substituted or unsubstituted C₁ to C₁₂ alkyl, unsubstituted or substituted C₆ to C₁₂ aryl, substituted or unsubstituted C₃ to C₁₂ heteroaryl; wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
- A³: is selected from H or D, preferably H.

According to one embodiment of the organic electronic device, wherein the Ce(IV) metal complex and/or Ce(IV) metal complex of formula (I) is selected from E1 to E17:

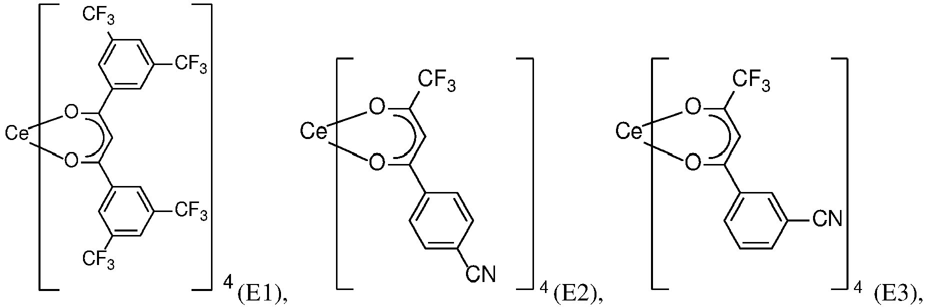

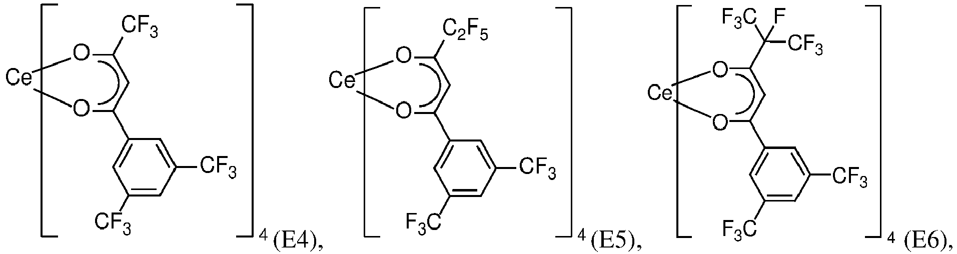

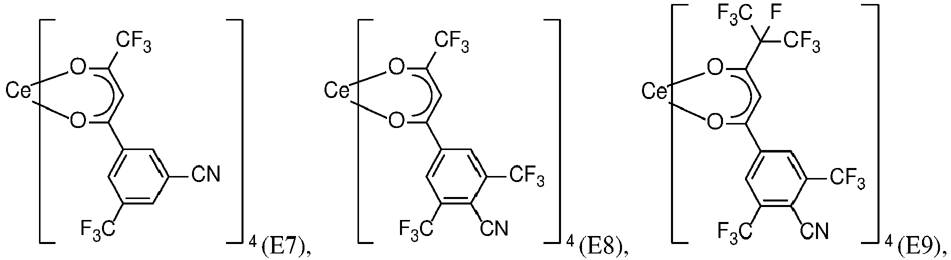

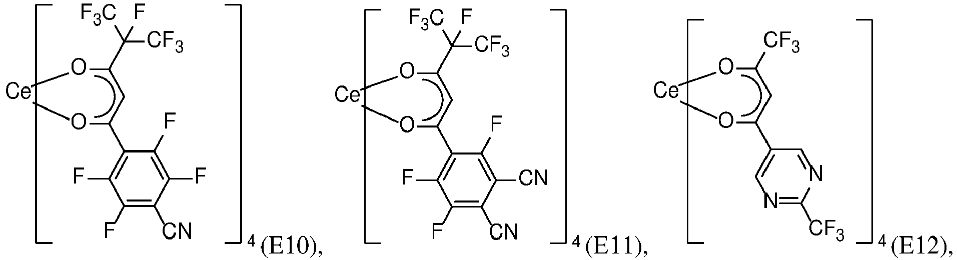

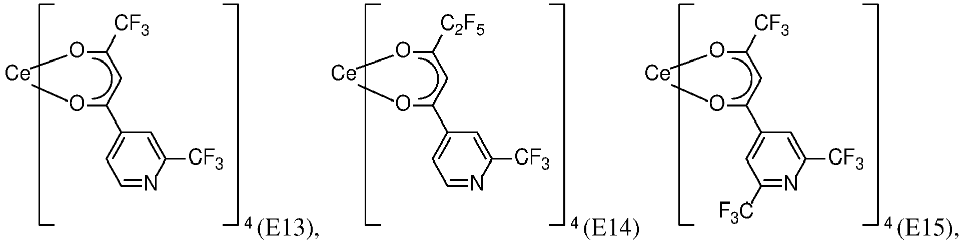

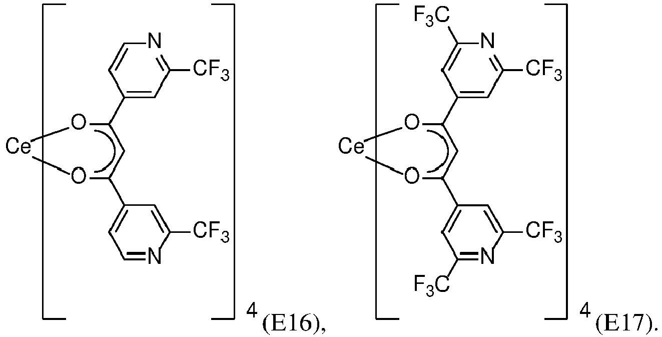

According to one embodiment, the Ce(IV) metal complex and/or Ce(IV) metal complex of formula (I) is preferably selected from (E2) to (E17) and/or selected from (E2) to (E15).

According to an embodiment, the following compounds E6, E7, E8, E9, E10, E15, and/or E16 may be excluded from the Ce(IV) metal complex and Ce(IV) metal complex of formula (I): 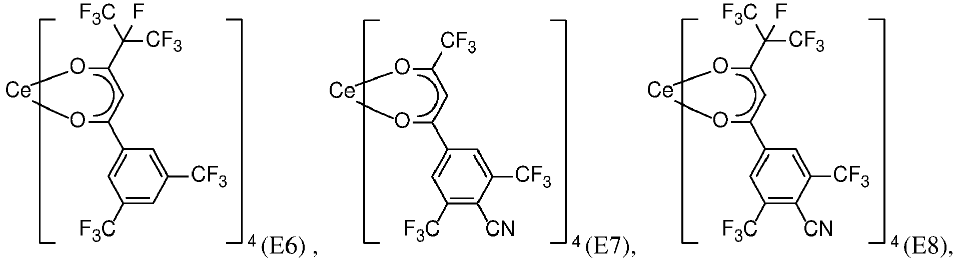 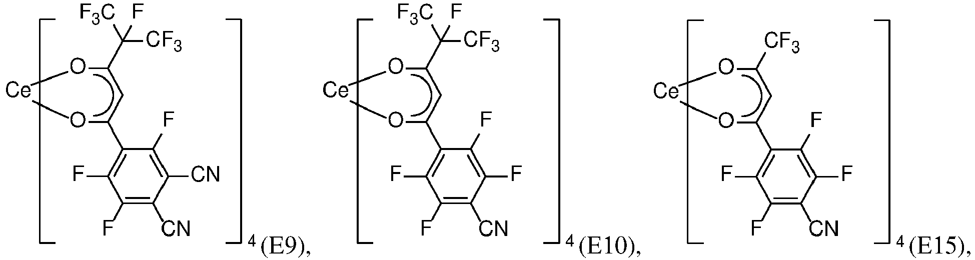 and/or

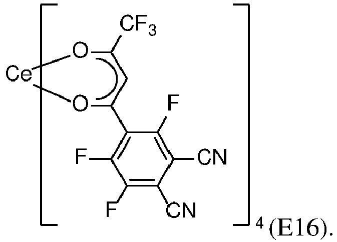

### Organic hole transport compound

According to one embodiment, the p-type charge generation layer comprises an organic hole transport compound, also referred to as "covalent organic matrix compound", "organic matrix compound" or "substantially covalent organic matrix compound", preferably the organic hole transport compound is a covalent organic matrix compound.

Preferred examples of the organic hole transport compounds are organic compounds consisting substantially of covalently bound C, H, O, N, S, which may optionally comprise also covalently bound B, P or Si.

In one embodiment, the organic hole transport compound is free of metal atoms and majority of its skeletal atoms may be selected from C, O, S, N. Alternatively, the organic hole transport compound is free of metal atoms and majority of its skeletal atoms may be selected from C and N.

According to one embodiment, the organic hole transport compound of the p-type charge generation layer may have a molecular weight Mw of ≥ 400 and ≤ 2000 g/mol, preferably a molecular weight Mw of ≥ 450 and ≤ 1500 g/mol, further preferred a molecular weight Mw of ≥ 500 and ≤ 1000 g/mol, in addition preferred a molecular weight Mw of ≥ 550 and ≤ 900 g/mol, also preferred a molecular weight Mw of ≥ 600 and ≤ 800 g/mol.

In one embodiment, the HOMO energy of the organic hole transport compound may be more negative than the HOMO energy of N2,N2,N2',N2',N7,N7,N7',N7'-octakis(4-methoxyphenyl)-9,9'-spirobi[fluorene]-2,2',7,7'-tetraamine (CAS 207739-72-8) when determined under the same conditions.

According to one embodiment of the present invention, the HOMO energy of the organic hole transport compound of the p-type charge generation layer may be calculated by quantum mechanical methods.

In one embodiment of the present invention, the organic hole transport compound may be free of alkoxy groups.

Preferably, the organic hole transport compound comprises at least one arylamine moiety, alternatively a diarylamine moiety, alternatively a triarylamine moiety.

Preferably, the organic hole transport compound of the p-type charge generation layer is free of metals and/or ionic bonds.

### Compound of formula (IIIa) or a compound of formula (IIIb)

According to one embodiment, wherein the organic hole transport compound of the p-type charge generation layer is selected from the group comprising arylamine compound, diarylamine compound, triarylamine compound, a compound of formula (IIIa) or a compound of formula (IIIb) .

According to another aspect of the present invention, the organic hole transport compound of the p-type charge generation layer, or the p-type charge generation layer and the hole transport layer may comprises at least one arylamine compound, diarylamine compound, triarylamine compound, a compound of formula (IIIa) or a compound of formula (IIIb): 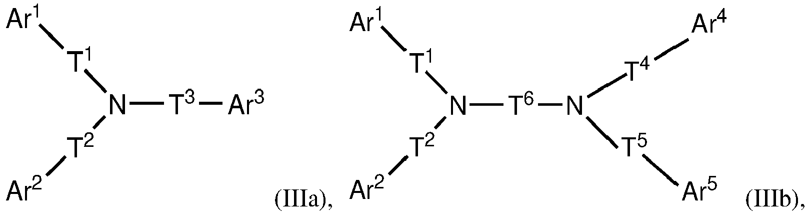 wherein:
- T¹, T², T³, T⁴ and T⁵: are independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R² , CN, Si(R²)₃, P(-O)(R²)₂, OR², S(-O)R² , S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

According to an embodiment of the organic electronic device, wherein the organic hole transport compound of the p-type charge generation layer, or the p-type charge generation layer and the hole transport layer may comprises a compound of formula (IIIa) or formula (IIIb): 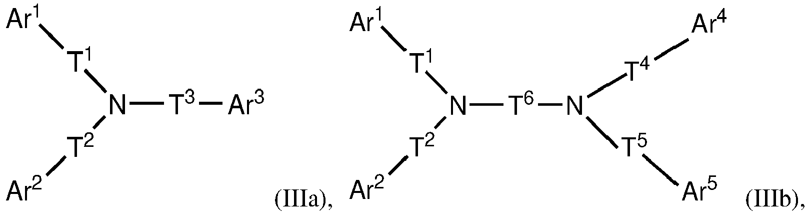 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein the substituents of Ar¹, Ar², Ar³, Ar⁴ and Ar⁵ are selected the same or different from the group comprising H, straight-chain alkyl having 1 to 20 carbon atoms, branched alkyl having 1 to 20 carbon atoms, cyclic alkyl having 3 to 20 carbon atoms, alkenyl or alkynyl groups having 2 to 20 carbon atoms, alkoxy groups having 1 to 20 carbon atoms, C₆ to C₁₈ aryl, C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle.

Preferably, the substituents of Ar¹, Ar², Ar³, Ar⁴ and Ar⁵ are selected the same or different from the group comprising H, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, alkoxy groups having 1 to 6 carbon atoms, C₆ to C₁₈ aryl, C₃ to C₁₈ heteroaryl, a fused ring system comprising 2 to 4 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle; more preferred the substituents are selected the same or different from the group consisting of H, straight-chain alkyl having 1 to 4 carbon atoms, branched alkyl having 1 to 4 carbon atoms, cyclic alkyl having 3 to 4 carbon atoms and/or phenyl.

Thereby, the compound of formula (IIIa) or (IIIb) may have a rate onset temperature suitable for mass production.

According to an embodiment of the organic electronic device, wherein the organic hole transport compound of the p-type charge generation layer, or the p-type charge generation layer and the hole transport layer may comprises a compound of formula (IIIa) or formula (IIIb): 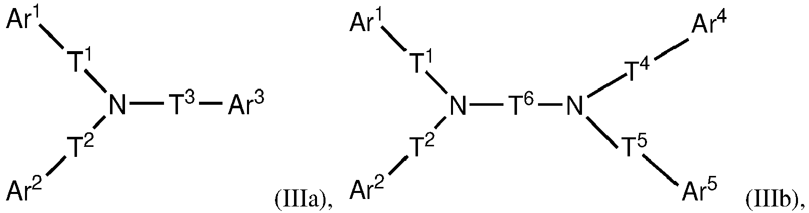 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene], or a unsubstituted aromatic fused ring system comprising at least three unsubstituted aromatic rings selected from the group comprising unsubstituted non-hetero, unsubstituted hetero 5-member rings, unsubstituted 6-member rings and/or unsubstituted 7-member rings, unsubstituted fluorene, or a fused ring system comprising 2 to 6 unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle.

According to an embodiment of the organic electronic device, wherein the organic hole transport compound of the p-type charge generation layer and/or hole transport layer comprises a compound of formula (IIIa) or formula (IIIb): 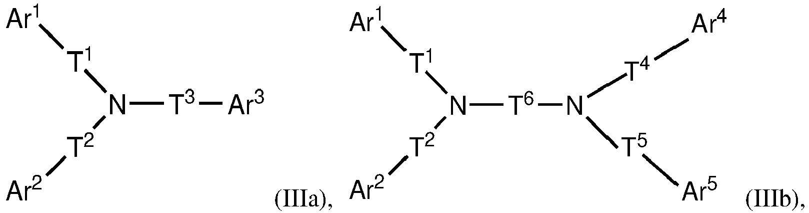 wherein
- T¹, T², T³, T⁴ and T⁵: may be independently selected from a single bond, phenylene, biphenylene, terphenylene or naphthenylene, preferably a single bond or phenylene;
- T⁶: is phenylene, biphenylene, terphenylene or naphthenylene;
- Ar¹, Ar², Ar³, Ar⁴ and Ar⁵: may be independently selected from unsubstituted C₆ to C₂₀ aryl, or unsubstituted C₃ to C₂₀ heteroarylene, unsubstituted biphenylene, unsubstituted fluorene, substituted 9-fluorene, substituted 9,9-fluorene, unsubstituted naphthalene, unsubstituted anthracene, unsubstituted phenanthrene, unsubstituted pyrene, unsubstituted perylene, unsubstituted triphenylene, unsubstituted tetracene, unsubstituted tetraphene, unsubstituted dibenzofurane, unsubstituted dibenzothiophene, unsubstituted xanthene, unsubstituted carbazole, substituted 9-phenylcarbazole, unsubstituted azepine, unsubstituted dibenzo[b,f]azepine, unsubstituted 9,9'-spirobi[fluorene], unsubstituted spiro[fluorene-9,9'-xanthene].

Thereby, the compound of formula (IIIa) or (IIIb) may have a rate onset temperature suitable for mass production.

According to an embodiment wherein T¹, T², T³, T⁴ and T⁵ may be independently selected from a single bond, phenylene, biphenylene or terphenylene. According to an embodiment wherein T¹, T², T³, T⁴ and T⁵ may be independently selected from phenylene, biphenylene or terphenylene and one of T¹, T², T³, T⁴ and T⁵ are a single bond. According to an embodiment wherein T¹, T², T³, T⁴ and T⁵ may be independently selected from phenylene or biphenylene and one of T¹, T², T³, T⁴ and T⁵ are a single bond. According to an embodiment wherein T¹, T², T³, T⁴ and T⁵ may be independently selected from phenylene or biphenylene and two of T¹, T², T³, T⁴ and T⁵ are a single bond.

According to an embodiment wherein T¹, T² and T³ may be independently selected from phenylene and one of T¹, T² and T³ are a single bond. According to an embodiment wherein T¹, T² and T³ may be independently selected from phenylene and two of T¹, T² and T³ are a single bond.

According to an embodiment wherein T⁶ may be phenylene, biphenylene, terphenylene. According to an embodiment wherein T⁶ may be phenylene. According to an embodiment wherein T⁶ may be biphenylene. According to an embodiment wherein T⁶ may be terphenylene.

According to an embodiment wherein Ar¹, Ar², Ar³, Ar⁴ and Ar⁵ may be independently selected from K1 to K16: 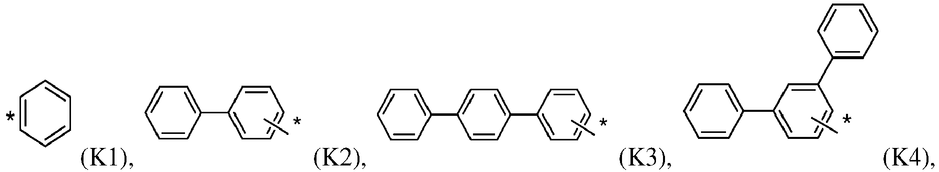 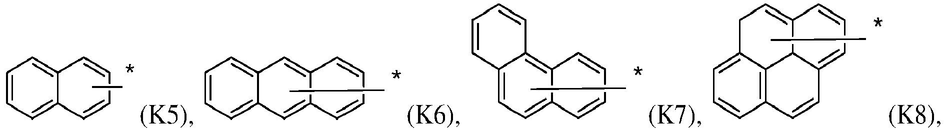 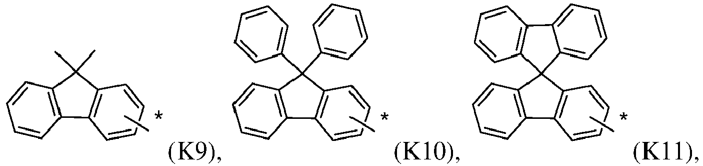 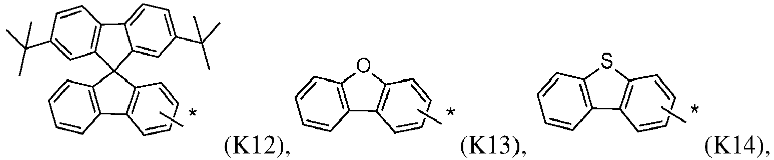 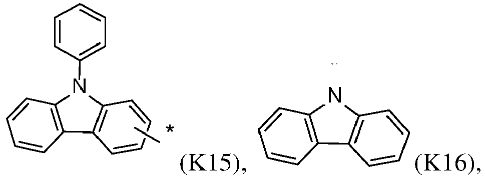 wherein the asterix "^{∗}" denotes the binding position.

According to an embodiment, wherein Ar¹, Ar², Ar³, Ar⁴ and Ar⁵ may be independently selected from K1 to K15; alternatively selected from K1 to K10 and K13 to K15.

According to an embodiment, wherein Ar¹, Ar², Ar³, Ar⁴ and Ar⁵ may be independently selected from the group consisting of K1, K2, K5, K7, K9, K10, K13 to K16.

The rate onset temperature may be in a range particularly suited to mass production, when Ar¹, Ar², Ar³, Ar⁴ and Ar⁵ are selected in this range.

According to one embodiment the organic hole transport compound of formula (IIIa) or formula (IIIb) may comprises at least ≥ 1 to ≤ 6 substituted or unsubstituted aromatic fused ring systems comprising heteroaromatic rings.

According to one embodiment the organic hole transport compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings.

According to one embodiment the organic hole transport compound of formula (IIIa) or formula (IIIb) may comprises at least ≥ 1 to ≤ 6 substituted or unsubstituted aromatic fused ring systems comprising heteroaromatic rings and at least ≥ 1 to ≤ 3 substituted or unsubstituted unsaturated 5- to 7-member ring of a heterocycle, preferably ≥ 2 to ≤ 5 substituted or unsubstituted aromatic fused ring systems comprising heteroaromatic rings, and at least ≥ 1 to ≤ 3 substituted or unsubstituted unsaturated 5- to 7-member ring of a heterocycle, further preferred 3 or 4 substituted or unsubstituted aromatic fused ring systems comprising heteroaromatic rings and optional at least ≥ 1 to ≤ 3 substituted or unsubstituted unsaturated 5- to 7-member ring of a heterocycle, and additional preferred wherein the aromatic fused ring systems comprising heteroaromatic rings are unsubstituted and optional at least ≥ 1 to ≤ 3 unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings.

According to one embodiment the organic hole transport compound of formula (IIIa) or formula (IIIb) may comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises at least ≥ 1 to ≤ 3 or 2 substituted or unsubstituted unsaturated 7-member ring of a heterocycle.

According to one embodiment substituted or unsubstituted aromatic fused ring systems of the organic hole transport compound of formula (IIIa) or formula (IIIb) may comprises at least ≥ 1 to ≤ 3 or 2 substituted or unsubstituted unsaturated 5- to 7-member ring of a heterocycle.

According to one embodiment the substituted or unsubstituted aromatic fused ring systems of the organic hole transport compound of formula (IIIa) or formula (IIIb) may comprises at least ≥ 1 to ≤ 3 or 2 substituted or unsubstituted unsaturated 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises substituted or unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises substituted or unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, and wherein the aromatic fused ring system comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises at least ≥ 1 to ≤ 6 substituted or unsubstituted aromatic fused ring systems, preferably ≥ 2 to ≤ 5 substituted or unsubstituted aromatic fused ring systems, and further preferred 3 or 4 substituted or unsubstituted aromatic fused ring systems, which comprises substituted or unsubstituted heteroaromatic rings, and wherein the aromatic fused ring system comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises:
- a substituted or unsubstituted aromatic fused ring systems with at least ≥ 2 to ≤ 6, preferably ≥ 3 to ≤ 5, or 4 fused aromatic rings selected from the group comprising substituted or unsubstituted non-hetero aromatic rings, substituted or unsubstituted hetero 5-member rings, substituted or unsubstituted 6-member rings and/or substituted or unsubstituted unsaturated 5-to 7- member ring of a heterocycle; or
- an unsubstituted aromatic fused ring systems with at least ≥ 2 to ≤ 6, preferably ≥ 3 to ≤ 5, or 4 fused aromatic rings selected from the group comprising unsubstituted non-hetero aromatic rings, unsubstituted hetero 5-member rings, unsubstituted 6-member rings and/or unsubstituted unsaturated 5- to 7-member ring of a heterocycle.

It should be noted here that the wording "aromatic fused ring system" may include at least one aromatic ring and at least one substituted or unsubstituted unsaturated 5- to 7- member ring. It should be noted here that the substituted or unsubstituted unsaturated 5- to 7- member ring may not be an aromatic ring.

According to one embodiment the organic hole transport compound of formula (IIIa) or formula (IIIb) may comprises at least at least ≥ 1 to ≤ 6, preferably ≥ 2 to ≤ 5, or further preferred 3 or 4 of the substituted or unsubstituted aromatic fused ring systems with:
- at least one unsaturated 5-member ring, and/or
- at least one unsaturated 6-member ring, and/or
- at least one unsaturated 7-member ring; wherein preferably at least one unsaturated 5- and/or at least one unsaturated 7-member ring comprises at least 1 to 3, preferably 1 hetero-atom.

According to one embodiment the organic hole transport compound of formula (IIIa) or formula (IIIb) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system comprises at least ≥ 1 to ≤ 3 or 2 substituted or unsubstituted unsaturated 5- to 7-member ring of a heterocycle.

According to one embodiment the organic hole transport compound of formula (IIIa) or formula (IIIb) may comprises :
- at least ≥ 6 to ≤ 12, preferably ≥ 7 to ≤ 11, further preferred ≥ 8 to ≤ 10 or 9 aromatic rings; and/or
- at least ≥ 4 to ≤ 11, preferably ≥ 5 to ≤ 10, further preferred ≥ 6 to ≤ 9 or in addition preferred 7 or 8 non-hetero aromatic rings, preferably the non-hetero aromatic rings are aromatic C₆ rings; and/or
- at least ≥ 1 to ≤ 4, preferably 2 or 3 aromatic 5-member-rings, preferably hetero aromatic 5-member-rings; and/or
- at least 1 or 2 unsaturated 5- to 7-member-ring of a heterocycle, preferably at least 1 or 2 unsaturated 7-member-ring of a heterocycle;
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and/or
- at least ≥ 6 to ≤ 12, preferably ≥ 7 to ≤ 11, further preferred ≥ 8 to ≤ 10 or 9 aromatic rings, wherein therefrom
   at least ≥ 4 to ≤ 11, preferably ≥ 5 to ≤ 10, further preferred ≥ 6 to ≤ 9 or in addition preferred 7 or 8 are non-hetero aromatic rings, and
   at least ≥ 1 to ≤ 4, preferably 2 or 3 aromatic rings are hetero aromatic rings, wherein the total number of non-hetero aromatic rings and hetero aromatic rings in total does not exceed 12 aromatic rings; and
   the hole transport compound or the hole transport compound according to formula I comprises at least ≥ 1 to ≤ 4, preferably 2 or 3 aromatic 5-member-rings, preferably hetero aromatic 5-member-rings, and/or
   the hole transport compound or the hole transport compound according to formula (I) comprises at least 1 or 2 unsaturated 5- to 7-member-ring of a heterocycle, preferably at least 1 or 2 unsaturated 7-member-ring of a heterocycle.

According to one embodiment the organic hole transport compound of formula (IIIa) or formula (IIIb) may comprises a hetero-atom, which may be selected from the group comprising O, S, N, B or P, preferably the hetero-atom may be selected from the group comprising O, S or N.

According to one embodiment the organic hole transport compound of formula (IIIa) or formula (IIIb) may comprises at least at least ≥ 1 to ≤ 6, preferably ≥ 2 to ≤ 5, or further preferred 3 or 4 of the substituted or unsubstituted aromatic fused ring systems with:
- at least one aromatic 5-member ring, and/or
- at least one aromatic 6-member ring, and/or
- at least one aromatic 7-member ring; wherein preferably at least one aromatic 5- and/or at least one aromatic 7-member ring comprises at least 1 to 3, preferably 1 hetero-atom;
wherein the substituted or unsubstituted aromatic fused ring system optional comprises at least ≥ 1 to ≤ 3 or 2 substituted or unsubstituted unsaturated 5- to 7-member ring of a heterocycle; and wherein the substituted or unsubstituted aromatic fused ring system comprises a hetero-atom, which may be selected from the group comprising O, S, N, B, P or Si, preferably the hetero-atom may be selected from the group comprising O, S or N.

According to one embodiment the organic hole transport compound of formula (IIIa) or formula (IIIb) may be free of hetero-atoms which are not part of an aromatic ring and/or part of an unsaturated 7-member-ring, preferably the hole transport compound or the hole transport compound according to formula (I) may be free on N-atoms except N-atoms which are part of an aromatic ring or are part of an unsaturated 7-member-ring.

According to an embodiment of the electronic device, wherein the compound of formula (IIIa) or formula (IIIb) are selected from F1 to F18:

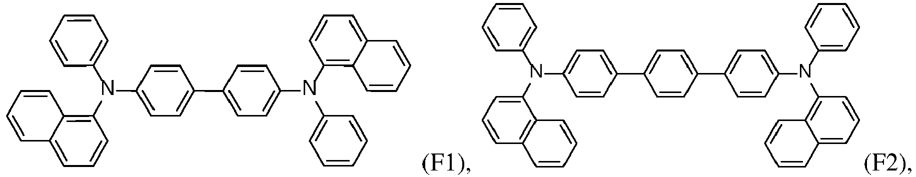

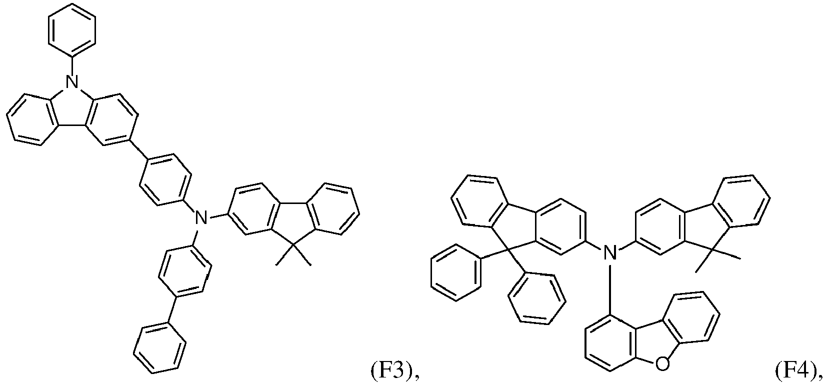

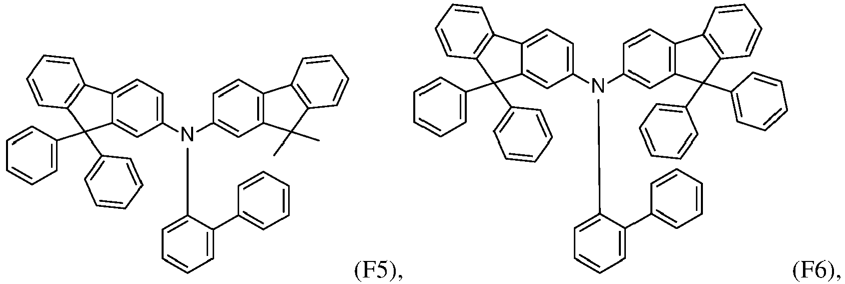

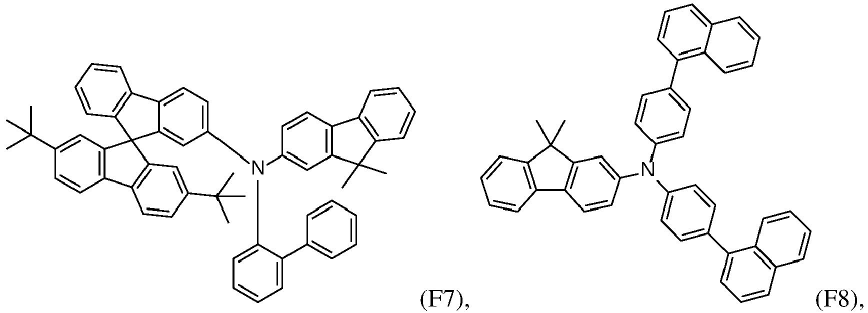

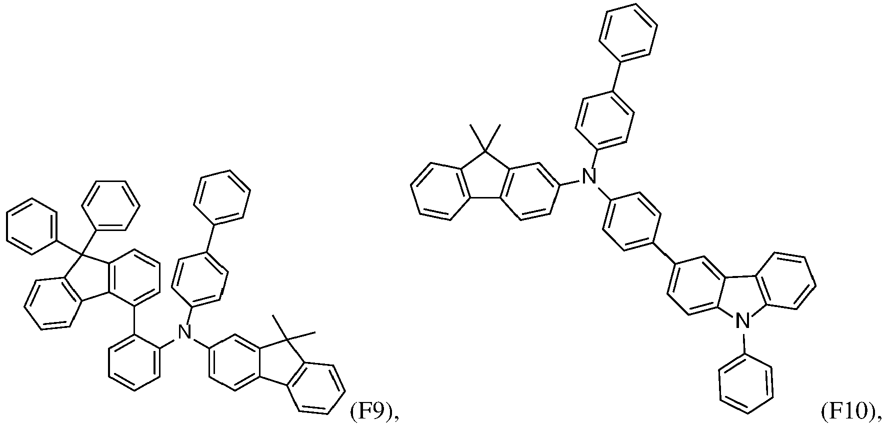

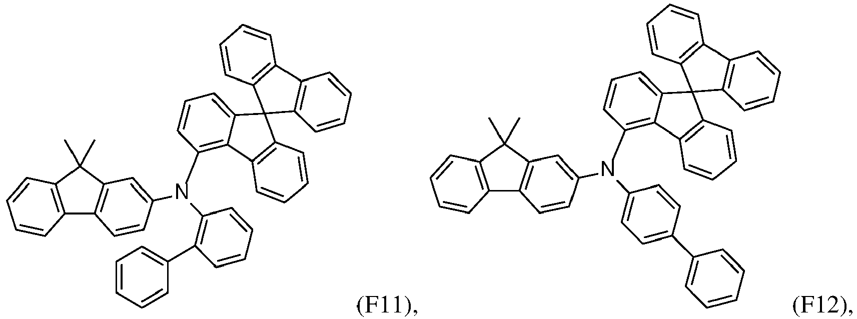

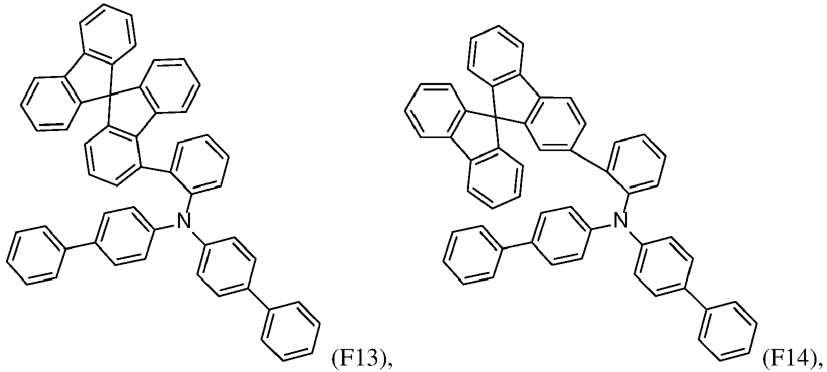

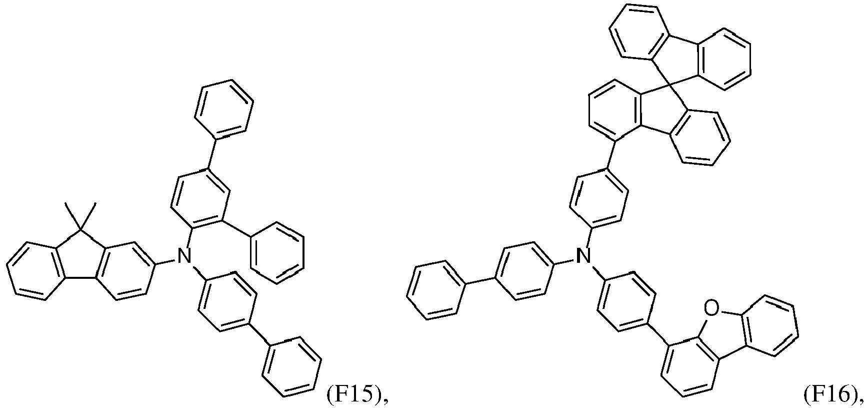

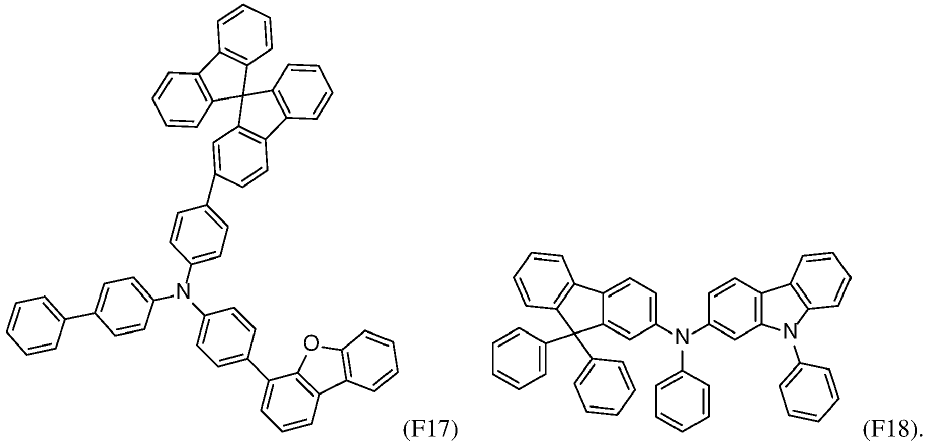

The organic hole transport compound may be free of HTM014, HTM081, HTM163, HTM222, EL-301, HTM226, HTM355, HTM133, HTM334, HTM604 and EL-22T. The abbreviations denote the manufacturers' names, for example, of Merck or Lumtec.

### p-type charge generation layer

The p-type charge generation layer may be formed on the n-type charge generation layer by vacuum deposition, spin coating, printing, casting, slot-die coating, Langmuir-Blodgett (LB) deposition, or the like. When the p-type charge generation layer is formed using vacuum deposition, the deposition conditions may vary according to the Ce(IV) metal complex and/or hole transport compound that is used to form the p-type charge generation layer, and the desired structure and thermal properties of the p-type charge generation layer. In general, however, conditions for vacuum deposition may include a deposition temperature of 100° C to 350° C, a pressure of 10⁻⁸ to 10⁻³ Torr (1 Torr equals 133.322 Pa), and a deposition rate of 0.1 to 10 nm/sec.

When the p-type charge generation layer is formed using spin coating or printing, coating conditions may vary according to the hole transport compound that is used to form the p-type charge generation layer, and the desired structure and thermal properties of the p-type charge generation layer. For example, the coating conditions may include a coating speed of about 2000 rpm to about 5000 rpm, and a thermal treatment temperature of about 80° C to about 200° C. Thermal treatment removes a solvent after the coating is performed.

The p-type charge generation layer may be formed of a Ce(IV) metal complex or Ce(IV) metal complex of formula (I) and an organic hole transport compound, wherein the organic hole transport compound is optionally a compound of formulae (IIIa) or (IIIb).

The thickness of the p-type charge generation layer may be in the range from about 5 nm to about 25 nm, and for example, from about 10 nm to about 15 nm.

When the thickness of the p-type charge generation layer is within this range, the p-type charge generation layer may have excellent hole generation characteristics, without a substantial penalty in operating voltage.

According to one embodiment of the present invention, the p-type charge generation layer may comprise:
- at least about ≥ 1 wt.-% to about ≤ 50 wt.-%, preferably about ≥ 5 wt.-% to about ≤ 40 wt.-%, and more preferred about ≥ 5 wt.-% to about ≤ 30 wt.-% of a Ce(IV) metal complex or Ce(IV) metal complex of formula (I), and
- at least about ≥ 50 wt.-% to about ≤ 99 wt.-%, preferably about ≥ 60 wt.-% to about ≤ 95 wt.-%, and more preferred about ≥ 95 wt.-% to about ≤ 70 wt.-% of an organic hole transport compound, preferably an organic hole transport compound of formulae (IIIa) or (IIIb); preferably the wt.-% of the Ce(IV) metal complex or Ce(IV) metal complex of formula (I) is lower than the wt.-% of the organic hole transport compound or an organic hole transport compound according to formulae (IIIa) or (IIIb); wherein the weight-% of the components are based on the total weight of the p-type charge generation layer.

Preferably, the p-type charge generation layer is free of ionic liquids, metal fluoride and/or metal oxides, wherein the metal in the metal oxide is selected from Re and/or Mo. Thereby, the p-type charge generation layer can be deposited under conditions suitable for mass production.

According to an embodiment of the organic electronic device, wherein the p-type charge generation layer is non-emissive.

It is to be understood that the p-type charge generation layer is not part of the n-type charge generation layer or hole transport layer.

### Organic electron transport compound

According to an embodiment of the present invention, the organic electron transport compound of the n-type charge generation layer may comprise at least one C₂ to C₂₄ N-heteroaryl or P=X group, with X being O, P, Se.

According to an embodiment of the present invention, the at least one C₂ to C₂₄ N-heteroaryl may be selected from a compound comprising at least one azine group, preferably at least two azine groups, also preferred three azine groups.

According to an embodiment of the present invention, the n-type charge generation layer may comprise an organic electron transport compound comprising at least one group selected from the list consisting of pyridine, pyrimidine, triazine, imidazole, benzimidazole, benzooxazole, quinone, benzoquinone, quinoxaline, benzoquinoxaline, acridine, phenanthroline, benzoacridine, dibenzoacridine.

According to an embodiment of the present invention, the n-type charge generation layer may comprise an organic electron transport compound comprising at least one phenanthroline group, preferably two phenanthroline groups.

According to an embodiment of the present invention, the organic electron transport compound of the n-type charge generation layer may comprise at least one P=O group.

According to another embodiment of the present invention, the organic electron transport compound differs in its chemical formula from the organic hole transport compound.

### Metal dopant

The metal dopant of the n-type charge generation layer may be selected from a metal with an electronegativity of ≤1.4 eV by Pauling scale. The metal dopant may be preferably selected from a metal with an electronegativity of ≤1.35 eV by Pauling scale. More preferred is metal dopant having an electronegativity of ≤1.4 eV by Pauling scale, wherein the metal dopant is selected from Li, Na, K, Rb, Cs, Mg, Ca, Sr, Ba, Sm, Eu or Yb, more preferred from Li, Na, K, Cs, Mg, Ca, Ba, Sm, Eu or Yb, even more preferred from Li, Mg or Yb. According to an embodiment, the metal dopant is not a Ce(IV) metal complex.

### n-type charge generation layer

The thickness of the n-type charge generation may be in the range from about 5 nm to about 25 nm, for example, in the range from about 10 nm to about 20 nm. When the thickness of the n-type charge generation layer is within this range, the n-type charge generation layer may have satisfactory electron-generating properties, without a substantial penalty in operating voltage.

According to one embodiment, the n-type charge generation layer may comprise
- at least about ≥ 0.5 to about ≤ 10 vol.-%, preferably about ≥ 1 to about ≤ 5 vol.-% metal dopant; and
- at least about ≤ 99.5 vol.-% to about ≥ 90 vol.-%, preferably ≤ 99 vol.-% to about ≥ 95 vol.-% organic electron transport compound.

### Charge generation layer

According to one embodiment, the n-type charge generation layer and the p-type charge generation layer may be arranged in direct contact.

According to one embodiment of the present invention, the p-type and/or n-type charge generation layer and/or the Ce(IV) metal complex are non-emissive.

In the context of the present specification the term "essentially non-emissive" or "non-emissive" means that the contribution of organic electron transport compound, the organic hole transport compound, the organic hole transport compound of formula (IIIa) or formula (IIIb), the Ce(IV) metal complex and/or layer, the p-type charge generation layer, as well as the n-type charge generation layer, to the visible emission spectrum from an organic electronic device, such as OLED or display device, is less than 10 %, preferably less than 5 % relative to the visible emission spectrum. The visible emission spectrum is an emission spectrum with a wavelength of about ≥ 380 nm to about ≤ 780 nm.

### Charge generation connecting layer

According to one embodiment, the charge generation layer (CGL) may further comprise a charge generation connecting layer (c-CGL), wherein the charge generation connecting layer is arranged between the n-type charge generation layer and the p-type charge generation layer. Preferably, the charge generation connecting layer is in direct contact with the n-type charge generation layer and the p-type charge generation layer.

The charge generation connecting layer may comprise or consist of a conductive compound.

According to an embodiment, the conductive compound is selected from HAT-CN or a metal complex, preferably metal phthalocyanine, zinc phthalocyanine (ZnPc) or copper phthalocyanine (CuPc).

The thickness of the charge generation connecting layer may be selected between about 0.5 and 5 nm, preferably 1 and 2.5 nm.

### Further layers

In accordance with the invention, the organic electronic device may comprise, besides the layers already mentioned above, further layers. Exemplary embodiments of respective layers are described in the following:

### Substrate

The substrate may be any substrate that is commonly used in manufacturing of, electronic devices, such as organic light-emitting diodes. If light is to be emitted through the substrate, the substrate shall be a transparent or semitransparent material, for example a glass substrate or a transparent plastic substrate. If light is to be emitted through the top surface, the substrate may be both a transparent as well as a non-transparent material, for example a glass substrate, a plastic substrate, a metal substrate, a silicon substrate or a transistor backplane. Preferably, the substrate is a silicon substrate or transistor backplane.

### Anode layer

The anode layer may be formed by depositing or sputtering a material that is used to form the anode layer. The material used to form the anode layer may be a high work-function material, so as to facilitate hole injection. The anode material may also be selected from a low work function material (i.e. aluminum). The anode layer may be a transparent or reflective electrode. Transparent conductive oxides, such as indium tin oxide (ITO), indium zinc oxide (IZO), tin-dioxide (SnO2), aluminum zinc oxide (AlZO) and zinc oxide (ZnO), may be used to form the anode layer. The anode layer may also be formed using metals, typically silver (Ag), gold (Au), or metal alloys.

According to one embodiment, the anode layer may be formed on a substrate, preferably the anode layer is in direct contact with a substrate.

### Hole injection layer

A hole injection layer (HIL) may be formed on the anode layer by vacuum deposition, spin coating, printing, casting, slot-die coating, Langmuir-Blodgett (LB) deposition, or the like. When the HIL is formed using vacuum deposition, the deposition conditions may vary according to the compound that is used to form the HIL, and the desired structure and thermal properties of the HIL. In general, however, conditions for vacuum deposition may include a deposition temperature of 100° C to 500° C, a pressure of 10⁻⁸ to 10⁻³ Torr (1 Torr equals 133.322 Pa), and a deposition rate of 0.1 to 10 nm/sec.

When the HIL is formed using spin coating or printing, coating conditions may vary according to the compound that is used to form the HIL, and the desired structure and thermal properties of the HIL. For example, the coating conditions may include a coating speed of about 2000 rpm to about 5000 rpm, and a thermal treatment temperature of about 80° C to about 200° C. Thermal treatment removes a solvent after the coating is performed.

The HIL may be formed of any compound that is commonly used to form a HIL. Examples of compounds that may be used to form the HIL include a phthalocyanine compound, such as copper phthalocyanine (CuPc), 4,4',4"-tris (3-methylphenylphenylamino) triphenylamine (m-MTDATA), TDATA, 2T-NATA, polyaniline/dodecylbenzenesulfonic acid (Pani/DBSA), poly(3,4-ethylenedioxythiophene)/poly(4-styrenesulfonate) (PEDOT/PSS), polyaniline/camphor sulfonic acid (Pani/CSA), and polyaniline)/poly(4-styrenesulfonate (PANI/PSS).

The HIL may comprise or consist of p-type dopant and the p-type dopant may be selected from tetrafluoro-tetracyanoquinonedimethane (F4TCNQ), 2,2'-(perfluoronaphthalen-2,6-diylidene) dimalononitrile or 2,2',2"-(cyclopropane-1,2,3-triylidene)tris(2-(p-cyanotetrafluorophenyl)acetonitrile) but not limited hereto.

According to an embodiment, the p-type dopant is selected from a radialene compound, for example 2,2',2"-(cyclopropane-1,2,3-triylidene)tris(2-(p-cyanotetrafluorophenyl)acetonitrile).

The HIL may comprise an organic hole transport compound and a p-type dopant.

The p-type dopant concentrations can be selected from 1 to 50 wt.-%, more preferably from 3 wt.-% to 30 wt.-%.

The p-type dopant concentrations can be selected from 1 to 50 vol.-%, more preferably from 3 vol.-% to 30 vol.-%.

According to a preferred embodiment of the present invention, however, the HIL comprises a Ce(IV) metal complex or a Ce(IV) metal complex of formula (I) as described above.

According to a preferred embodiment of the present invention, the HIL may comprises the same Ce(IV) metal complex or a Ce(IV) metal complex of formula (I) as in the p-type charge generation layer.

According to a preferred embodiment of the present invention, the HIL may comprises an organic hole transport compound as described above.

According to a preferred embodiment of the present invention, the HIL may comprises a Ce(IV) metal complex or a Ce(IV) metal complex of formula (I), as described above, and an organic hole transport compound or a compound of formula (IIIa) or (IIIb), as described above.

According to a preferred embodiment of the present invention, the p-type charge generation layer and the hole injection layer may comprise an identical organic hole transport compound or compound of formula (IIIa) or (IIIb).

The thickness of the HIL may be in the range from about 1 nm to about 100 nm, and for example, from about 1 nm to about 25 nm. When the thickness of the HIL is within this range, the HIL may have excellent hole injecting characteristics, without a substantial penalty in operating voltage.

### Hole transport layer

A hole transport layer (HTL) may be formed on the HIL by vacuum deposition, spin coating, slot-die coating, printing, casting, Langmuir-Blodgett (LB) deposition, or the like. When the HTL is formed by vacuum deposition or spin coating, the conditions for deposition and coating may be similar to those for the formation of the HIL. However, the conditions for the vacuum or solution deposition may vary, according to the compound that is used to form the HTL.

According to one embodiment of the present invention, the hole transport layer may comprise an organic hole transport compound as described above.

According to a preferred embodiment of the present invention, the hole injection layer and the hole transport layer may comprise an identical organic hole transport compound as described above.

According to one embodiment the p-type charge generation layer and the hole transport layer comprises an organic hole transport compound.

According to one embodiment the p-type charge generation layer and the hole transport layer comprise an organic hole transport compound, wherein the organic hole transport compound in the p-type charge generation layer and in the hole transport layer are selected the same.

According to one embodiment of the present invention, the hole transport layer may comprise a compound of formula (IIIa) or (IIIb) as described above.

According to a preferred embodiment of the present invention, the hole injection layer and the hole transport layer may comprise an identical compound of formula (IIIa) or (IIIb) as described above.

According to a preferred embodiment of the present invention, the p-type charge generation layer, the hole injection layer and the hole transport layer may comprise an identical organic hole transport compound.

According to a preferred embodiment of the present invention, the p-type charge generation layer, the hole injection layer and the hole transport layer may comprise an identical compound of formula (IIIa) or (IIIb) as described above.

According to a preferred embodiment of the present invention, the hole injection layer, the hole transport layer and the p-type charge generation layer may comprise an identical compound of formula (IIIa) or (IIIb) as described above; and the hole injection layer and the p-type charge generation layer may comprise an identical Ce(IV) metal complex or Ce(IV) metal complex of formula (I), as described above.

The thickness of the HTL may be in the range of about 5 nm to about 250 nm, preferably, about 10 nm to about 200 nm, further about 20 nm to about 190 nm, further about 40 nm to about 180 nm, further about 60 nm to about 170 nm, further about 80 nm to about 160 nm, further about 100 nm to about 160 nm, further about 120 nm to about 140 nm. A preferred thickness of the HTL may be 170 nm to 200 nm.

When the thickness of the HTL is within this range, the HTL may have excellent hole transporting characteristics, without a substantial penalty in operating voltage.

### Electron blocking layer

The function of an electron blocking layer (EBL) is to prevent electrons from being transferred from an emission layer to the hole transport layer and thereby confine electrons to the emission layer. Thereby, efficiency, operating voltage and/or lifetime may be improved. Typically, the electron blocking layer comprises a triarylamine compound.

If the electron blocking layer has a high triplet level, it may also be described as triplet control layer.

The function of the triplet control layer is to reduce quenching of triplets if a phosphorescent green or blue emission layer is used. Thereby, higher efficiency of light emission from a phosphorescent emission layer can be achieved. The triplet control layer may be selected from triarylamine compounds with a triplet level above the triplet level of the phosphorescent emitter in the adjacent emission layer.

The thickness of the electron blocking layer may be selected between 2 and 20 nm.

### Emission layer (EML)

The at least one first emission layer (EML), also referred to as first emission layer may be formed on the HTL or EBL by vacuum deposition, spin coating, slot-die coating, printing, casting, LB deposition, or the like. When the EML is formed using vacuum deposition or spin coating, the conditions for deposition and coating may be similar to those for the formation of the p-type charge generation layer. However, the conditions for deposition and coating may vary, according to the compound that is used to form the EML.

According to the present invention it is preferred that the organic electronic device comprises one emission layer that is named "first emission layer". However the organic electronic device optionally comprises two emission layers, wherein the first emission layer is named first emission layer and second emission layer is named second emission layer.

The at least one emission layer (EML) may be formed of a combination of a host and an emitter dopant. Example of the host are Alq3, 4,4'-N,N'-dicarbazole-biphenyl (HTC-10), poly(n-vinyl carbazole) (PVK), 9,10-di(naphthalene-2-yl)anthracene (ADN), 4,4',4"-tris(carbazol-9-yl)-triphenylamine(TCTA), 1,3,5-tris(N-phenylbenzimidazole-2-yl)benzene (TPBI), 3-tert-butyl-9,10-di-2-naphthylanthracenee (TBADN), distyrylarylene (DSA) and bis(2-(2-hydroxyphenyl)benzo-thiazolate)zinc (Zn(BTZ)2).

The emitter dopant may be a phosphorescent or fluorescent emitter. Phosphorescent emitters and emitters which emit light via a thermally activated delayed fluorescence (TADF) mechanism may be preferred due to their higher efficiency. The emitter may be a small molecule or a polymer.

Examples of red emitter dopants are PtOEP, Ir(piq)3, and Btp21r(acac), but are not limited thereto. These compounds are phosphorescent emitters, however, fluorescent red emitter dopants could also be used.

Examples of phosphorescent green emitter dopants are Ir(ppy)3 (ppy = phenylpyridine), Ir(ppy)2(acac), Ir(mpyp)3.

Examples of phosphorescent blue emitter dopants are F21rpic, (F2ppy)₂Ir(tmd) and Ir(dfppz)3 and ter-fluorene. 4.4'-bis(4-diphenyl amiostyryl)biphenyl (DPAVBi), 2,5,8,11-tetra-tert-butyl perylene (TBPe) are examples of fluorescent blue emitter dopants.

The amount of the emitter dopant may be in the range from about 0.01 to about 50 parts by weight, based on 100 parts by weight of the host. Alternatively, the at least one emission layer may consist of a light-emitting polymer. The EML may have a thickness of about 10 nm to about 100 nm, for example, from about 20 nm to about 60 nm. When the thickness of the EML is within this range, the EML may have excellent light emission, without a substantial penalty in operating voltage.

It may be provided that the at least one emission layer also referred to as first emission layer is free of the Ce(IV) metal complex or Ce(IV) metal complex of formula (I) of the p-type charge generation layer.

It may be provided that the at least one emission layer does not comprise the compound of Formulae (IIIa) or (IIIb).

### Hole blocking layer (HBL)

A hole blocking layer (HBL) may be formed on the EML, by using vacuum deposition, spin coating, slot-die coating, printing, casting, LB deposition, or the like, in order to prevent the diffusion of holes into the n-type charge generation. When the EML comprises a phosphorescent emitter dopant, the HBL may have also a triplet exciton blocking function.

When the HBL is formed using vacuum deposition or spin coating, the conditions for deposition and coating may be similar to those for the formation of the p-type charge generation layer. However, the conditions for deposition and coating may vary, according to the compound that is used to form the HBL. Any compound that is commonly used to form a HBL may be used. Examples of compounds for forming the HBL include oxadiazole derivatives, triazole derivatives, phenanthroline derivatives and triazine derivatives.

The HBL may have a thickness in the range from about 5 nm to about 100 nm, for example, from about 10 nm to about 30 nm. When the thickness of the HBL is within this range, the HBL may have excellent hole-blocking properties, without a substantial penalty in driving voltage.

### Electron transport layer (ETL)

The organic electronic device according to the present invention may further comprise an electron transport layer (ETL).

According to another embodiment of the present invention, the electron transport layer may comprise at least one compound selected from the group comprising an azine, triazine, phenanthroline, imidazole, benzimidazole and/or phosphine oxide compound, and preferably a phosphine oxide compound, and preferably an azine compound, or triazine compound, and more preferred a phosphine oxide compound.

In one embodiment, the may comprise a dopant selected from an organic metal complex, preferably an alkali organic complex, preferably LiQ.

The thickness of the ETL may be in the range from about 15 nm to about 50 nm, for example, in the range from about 20 nm to about 40 nm.

### Electron injection layer (EIL)

An optional EIL, which may facilitates injection of electrons from the cathode, may be formed on the electron transport layer (ETL), preferably directly on the ETL . Examples of materials for forming the EIL include lithium 8-hydroxyquinolinolate (LiQ), LiF, NaCl, CsF, Li20, BaO, Ca, Ba, Yb, Mg which are known in the art. Deposition and coating conditions for forming the EIL are similar to those for formation of the n-type charge generation layer, although the deposition and coating conditions may vary, according to the material that is used to form the EIL.

The thickness of the EIL may be in the range from about 0.1 nm to about 30 nm, for example, in the range from about 0.5 nm to about 25 nm. When the thickness of the EIL is within this range, the EIL may have satisfactory electron-injecting properties, without a substantial penalty in operating voltage.

### Cathode layer

The cathode layer is formed on the p-type charge generation layer, ETL or optional EIL if present. The cathode layer may be formed of a metal, an alloy, an electrically conductive compound, or a mixture thereof. The cathode layer may have a low work function. For example, the cathode layer may be formed of lithium (Li), magnesium (Mg), aluminum (Al), aluminum (Al)-lithium (Li), calcium (Ca), barium (Ba), ytterbium (Yb), magnesium (Mg)-indium (In), magnesium (Mg)-silver (Ag), or the like. Alternatively, the cathode layer may be formed of a transparent conductive oxide, such as ITO or IZO.

The thickness of the cathode layer may be in the range from about 5 nm to about 1000 nm, for example, in the range from about 10 nm to about 100 nm. When the thickness of the cathode layer is in the range from about 5 nm to about 50 nm, the cathode layer may be transparent or semitransparent even if formed from a metal or metal alloy.

It is to be understood that the cathode layer is not part of an electron injection layer, second HTL and/or the p-type charge generation layer.

### Organic electronic device

According to one embodiment, the organic electronic device may further comprise a substrate, wherein the anode layer or the cathode layer is arranged on the substrate; preferably the anode layer or cathode layer is arranged in direct contact with the substrate.

According to one embodiment, the organic electronic device may further comprise a hole injection layer, wherein the hole injection layer is arranged between the anode layer and the n-type charge generation layer.

According to one embodiment, the organic electronic device may further comprise a first hole transport layer; wherein the first hole transport layer is arranged between the anode layer and the n-type charge generation layer.

According to one embodiment, the organic electronic device may further comprise a hole injection layer and a first hole transport layer; wherein the first hole transport layer is arranged between the hole injection layer and the n-type charge generation layer.

According to one embodiment, the organic electronic device may further comprise a first hole transport layer and a second hole transport layer, wherein the first hole transport layer is arranged between the anode layer and the n-type charge generation layer and the second hole transport layer is arranged between p-type charge generation layer and the cathode layer.

According to one embodiment, wherein the first and/or second hole transport layer comprises an organic hole transport compound.

According to one embodiment, wherein the hole transport layer comprises an organic hole transport compound and the p-type charge generation layer comprises an organic hole transport compound, wherein the organic hole transport compound in the p-type charge generation layer and hole transport layer are selected the same.

According to one embodiment, wherein the first and second hole transport layer comprise an organic hole transport compound and the p-type charge generation layer comprises an organic hole transport compound wherein the organic hole transport compound in the p-type charge generation layer and the first and second hole transport layer are selected the same.

According to one embodiment, wherein the first and second hole transport layer comprise an organic hole transport compound and the p-type charge generation layer comprises an organic hole transport compound wherein the organic hole transport compound in the p-type charge generation layer and the first and second hole transport layer are selected differently thereto.

According to one embodiment, the organic electronic device may further comprise at least one first emission layer, wherein the at least one first emission layer is arranged between the anode layer and the n-type charge generation layer.

According to one embodiment, the organic electronic device may further comprise a first and a second emission layer, wherein the first emission layer is arranged between the anode layer and the n-type charge generation layer and the second emission layer is arranged between the p-type charge generation layer and the cathode layer.

The organic electronic device according to the invention may be an organic light-emitting diode.

According to one aspect of the present invention, there is provided an organic light-emitting diode (OLED) comprising: a substrate; an anode layer formed on the substrate; a charge generation layer according to invention, at least one emission layer and a cathode layer.

According to one aspect of the present invention, there is provided an organic light-emitting diode (OLED) comprising: a substrate; an anode layer formed on the substrate; a charge generation layer according to invention, at least a first and a second emission layers and a cathode layer, wherein the charge generation layer is arranged between the first and the second emission layer.

According to one aspect of the present invention, there is provided an organic light-emitting diode (OLED) comprising: a substrate; an anode layer formed on the substrate; a hole injection layer which may comprise a Ce(IV) metal complex or a Ce(IV) metal complex of formula (I), a hole transport layer, a first emission layer, an electron transport layer, a n-type charge generation layer, a p-type charge generation layer comprising a Ce(IV) metal complex or a Ce(IV) metal complex of formula (I), a hole transport layer, a second emission layer, an optional electron transport layer, an optional electron injection layer and a cathode layer.

According to various embodiments of the present invention, there may be provided OLEDs layers arranged between the above mentioned layers, on the substrate or on the top electrode.

According to one aspect, the OLED may comprise a layer structure of a substrate that is adjacent arranged to an anode electrode, the anode electrode is adjacent arranged to a first hole injection layer, the first hole injection layer is adjacent arranged to a first hole transport layer, the first hole transport layer is adjacent arranged to a first electron blocking layer, the first electron blocking layer is adjacent arranged to a first emission layer, the first emission layer is adjacent arranged to a first electron transport layer, the first electron transport layer is adjacent arranged to an n-type charge generation layer, the n-type charge generation layer is adjacent arranged to a p-type charge generating layer, the p-type charge generating layer is adjacent arranged to a second hole transport layer, the second hole transport layer is adjacent arranged to a second electron blocking layer, the second electron blocking layer is adjacent arranged to a second emission layer, between the second emission layer and the cathode electrode an optional electron transport layer and/or an optional electron injection layer are arranged.

### Method of manufacturing

According to another aspect of the present invention, there is provided a method of manufacturing an organic electronic device, the method using at least three deposition sources, preferably four deposition sources.

The methods for deposition that can be suitable comprise:
- deposition via vacuum thermal evaporation;
- deposition via solution processing, preferably the processing may be selected from spin-coating, printing, casting; and/or
- slot-die coating.

According to various embodiments of the present invention, there is provided a method using:
- a first deposition source to release the organic electronic transport compounds,
- a second deposition source to release the metal dopant,
- a third deposition source to release the organic hole transport compound, and
- a fourth deposition source to release the Ce(IV) metal complex or Ce(IV) metal complex of formula (I).

The method comprising
- the step of forming the n-type charge generation layer; whereby for an organic electronic device the n-type charge generation layer is formed by releasing the organic electron transport compound from the first deposition source and the metal dopant from the second deposition source;
- followed by a step of forming the p-type charge generation layer; whereby for an organic electronic device the p-type charge generation layer is formed by releasing the organic hole transport compound from the third deposition source and the Ce(IV) metal complex or Ce(IV) metal complex of formula (I) from the fourth deposition source; in this order or in the reverse order.

According to various embodiments of the present invention, the method may further include the steps for forming an organic electronic device, wherein in that order the layers substrate, anode layer, hole injection layer HIL, first hole transport layer HTL, first electron blocking layer EBL, first emission layer EML, first hole blocking layer HBL, first electron transport layer ETL, n-type charge generation, p-type charge generation layer, second hole transport layer HTL, second electron blocking layer, second emission layer, second hole blocking layer, second electron transport layer, electron injection layer, and cathode layer are formed on each other starting with a substrate onto an anode layer is formed; in this order or in the reverse order.

### Electronic device

According to another aspect, it is provided an electronic device comprising at least one organic light emitting device according to any embodiment described throughout this application, preferably, the electronic device comprises the organic light emitting diode in one of embodiments described throughout this application. More preferably, the organic electronic device is a light emitting device or a display device, preferably a display device.

### Ce(IV) metal complex of formula (Ia)

The invention further relates to a Ce(IV) metal complex of formula (Ia): 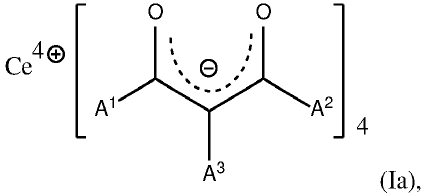 wherein
- A¹: is selected from formulae D1 and D4: 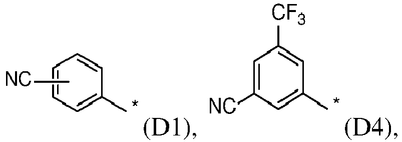
wherein the "^{∗}" denotes the binding position;
- A²: is selected from substituted or unsubstituted C₁ to C₁₂ alkyl, wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
- A³: is selected from H or D, preferably H; or
wherein
- A¹: is selected from formulae D39, D42 and D56: 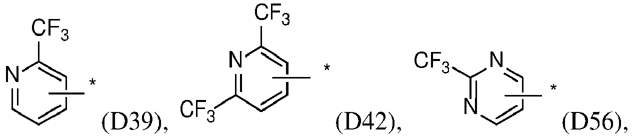
wherein the "^{∗}" denotes the binding position;
- A²: is selected from substituted or unsubstituted C₁ to C₁₂ alkyl, unsubstituted or substituted C₆ to C₁₂ aryl, substituted or unsubstituted C₃ to C₁₂ heteroaryl; wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
- A³: is selected from H or D, preferably H.

According to one embodiment of the Ce(IV) metal complex of formula (Ia), wherein the LUMO energy of the Ce(IV) metal complex or Ce(IV) metal complex of formula (Ia) is selected ≤ -4.65 eV and ≥ 6 eV, preferably ≤ -4.7 eV and ≥ 6 eV, also preferred ≤ -4.8 eV and ≥ 6 eV; wherein the LUMO energy is calculated as single point energy calculation with the program package TURBOMOLE V6.5 (TURBOMOLE GmbH, Litzenhardtstrasse 19, 76135 Karlsruhe, Germany) from the optimized geometry by applying the hybrid functional B3LYP with a Def-TZVP basis set in the gas phase and inclusion of dispersion forces as D3BJ. Geometry is optimized by applying the hybrid functional B3LYP with a Def-SVP basis set in the gas phase and inclusion of dispersion forces as D3BJ. If more than one conformation is viable, the conformation with the lowest total energy is selected

When the Ce(IV) metal complex of formula (Ia) is selected in this range, the LUMO energy may be particularly suited to a p-type charge generation layer, and/or the thermal stability and/or rate onset temperature may be in the range particularly suited to mass production.

According to another aspect of the present invention, a Ce(IV) metal complex of formula la: 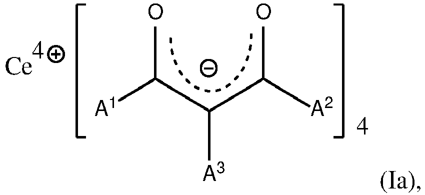 wherein
- A¹: is selected from formulae D1 and D4: 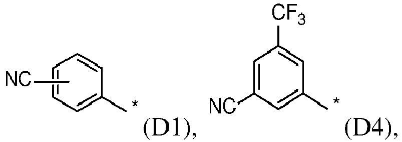
wherein the "^{∗}" denotes the binding position;
- A²: is selected from substituted or unsubstituted C₁ to C₆ alkyl, preferably C₁ to C₄ alkyl, wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
- A³: is selected from H or D, preferably H. According to another aspect of the invention, a Ce(IV) metal complex of formula la: 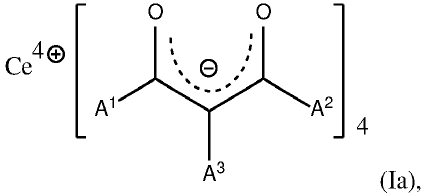
wherein
- A¹: is selected from formulae D39, D42 and D56: 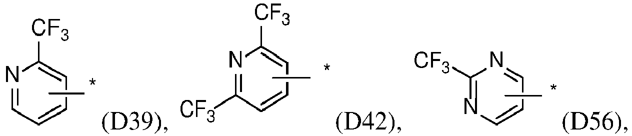
wherein the "^{∗}" denotes the binding position;
- A²: is selected from substituted or unsubstituted C₁ to C₆ alkyl, preferably C₁ to C₄ alkyl, unsubstituted or substituted phenyl, substituted or unsubstituted C₃ heteroaryl; wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and

- A³: is selected from H or D, preferably H.

According to another aspect of the invention, a Ce(IV) metal complex of formula la: 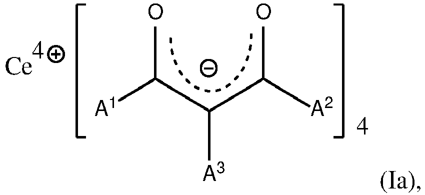 wherein
- A¹: is selected from formulae D39, D42 and D56: 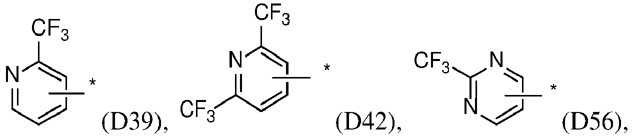
wherein the "^{∗}" denotes the binding position;
- A²: is selected from substituted or unsubstituted C₁ to C₆ alkyl, preferably C₁ to C₄ alkyl; wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
- A³: is selected from H or D, preferably H.

According to one embodiment, wherein the Ce(IV) metal complex of formula (Ia) is selected from E2 to E3, E7, or E12 to E17:

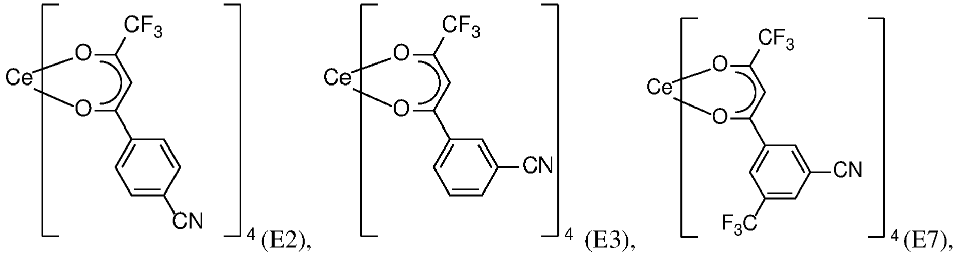

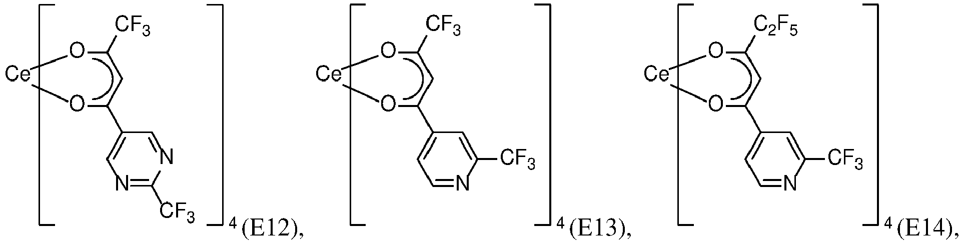

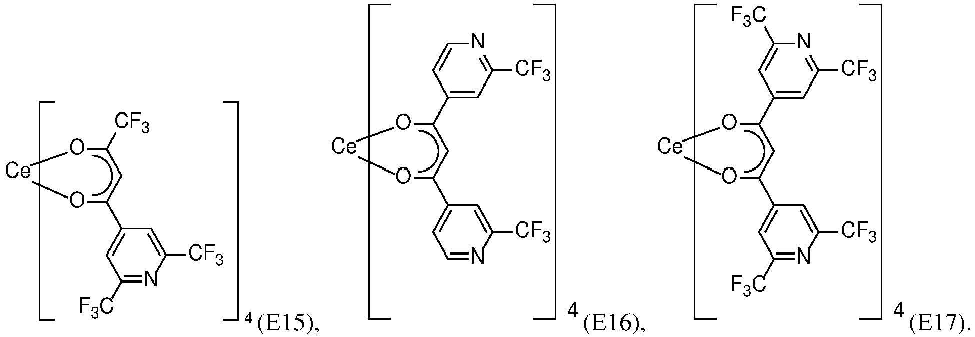

Hereinafter, the embodiments are illustrated in more detail with reference to examples. However, the present disclosure is not limited to the following examples. Reference will now be made in detail to the exemplary aspects.

### Description of the Drawings

The aforementioned components, as well as the claimed components and the components to be used in accordance with the invention in the described embodiments, are not subject to any special exceptions with respect to their size, shape, material selection and technical concept such that the selection criteria known in the pertinent field can be applied without limitations.

Additional details, characteristics and advantages of the object are disclosed in the dependent claims and the following description of the respective figures which in an exemplary fashion show preferred embodiments according to the invention. Any embodiment does not necessarily represent the full scope, however, and reference is made therefore to the claims and herein for interpreting the scope. It is to be understood that both the foregoing general description and the following detailed description are exemplary and explanatory only and are intended to provide further explanation of the present invention as claimed.

### Figures 1 to 5

- FIG. 1: is a schematic sectional view of an OLED comprising a charge generation layer, according to an exemplary embodiment of the present invention.
- FIG. 2: is a schematic sectional view of an OLED comprising a charge generation layer, according to an exemplary embodiment of the present invention.
- FIG. 3: is a schematic sectional view of a stacked OLED comprising a charge generation layer, according to an exemplary embodiment of the present invention.
- FIG. 4: is a schematic sectional view of a stacked OLED comprising a charge generation layer, according to an exemplary embodiment of the present invention.
- FIG. 5: is a schematic sectional view of a stacked OLED comprising a charge generation layer, according to an exemplary embodiment of the present invention.

Hereinafter, the figures are illustrated in more detail with reference to examples. However, the present disclosure is not limited to the following figures.

Herein, when a first element is referred to as being formed or disposed "on" or "onto" a second element, the first element can be disposed directly on the second element, or one or more other elements may be disposed there between. When a first element is referred to as being formed or disposed "directly on" or "directly onto" a second element, no other elements are disposed there between.

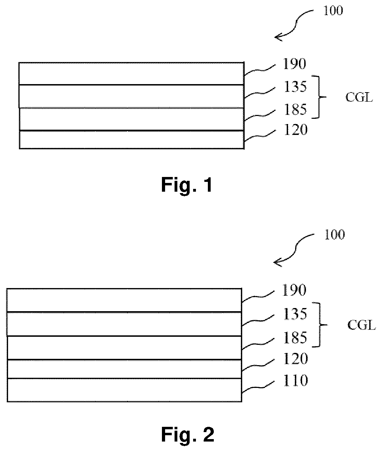

Fig. 1 is a schematic sectional view of an OLED 100, according to one exemplary embodiment of the present invention.

Referring to Fig. 1 the OLED 100 includes an anode layer 120, an n-type charge generation layer (n-CGL) 185, wherein the n-type charge generation layer (n-CGL) 185 comprises an electron transport compound and a metal dopant, a p-type charge generation layer (p-GCL) 135, wherein the p-type charge generation layer (p-GCL) 135 may comprise a Ce(IV) metal complex or a Ce(IV) metal complex of formula (I) and an organic hole transport compound, and a cathode layer 190.

Fig. 2 is a schematic sectional view of an OLED 100, according to one exemplary embodiment of the present invention.

Referring to Fig. 2 the OLED 100 includes a substrate 110, an anode layer 120, an n-type charge generation layer (n-CGL) 185, wherein the n-type charge generation layer (n-CGL) 185 comprises an electron transport compound and a metal dopant, a p-type charge generation layer (p-GCL) 135, wherein the p-type charge generation layer (p-GCL) 135 may comprise a Ce(IV) metal complex or a Ce(IV) metal complex of formula (I) and an organic hole transport compound, and a cathode layer 190.

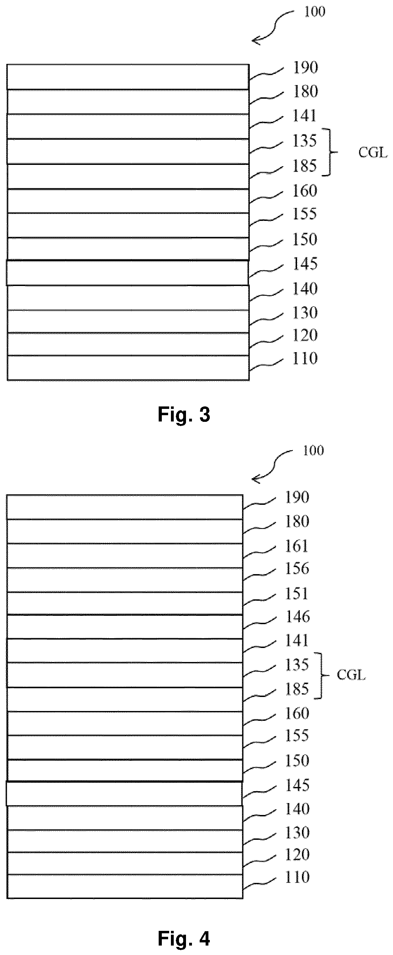

Fig. 3 is a schematic sectional view of an OLED 100, according to one exemplary embodiment of the present invention.

Referring to Fig. 3 the OLED 100 includes a substrate 110, an anode layer 120, a hole injection layer (HIL) 130, a first hole transport layer (HTL1) 140, an electron blocking layer (EBL) 145, an emission layer (EML) 150, a hole blocking layer (HBL) 155, an electron transport layer (ETL) 160, an n-type charge generation layer (n-CGL) 185, a p-type charge generation layer (p-GCL) 135, a second hole transport layer (HTL2) 141, and electron injection layer (EIL) 180 and a cathode layer 190. The HIL may comprise a Ce(IV) metal complex or a Ce(IV) metal complex of formula (I).

Fig. 4 is a schematic sectional view of a stacked OLED 100, according to another exemplary embodiment of the present invention. Fig. 4 differs from Fig. 3 in that the OLED 100 of Fig. 4 further comprises a second emission layer.

Referring to Fig. 4 the OLED 100 includes a substrate 110, an anode layer 120, a hole injection layer (HIL) 130, a first hole transport layer (HTL) 140, a first electron blocking layer (EBL) 145, a first emission layer (EML) 150, an optional first hole blocking layer (HBL) 155, a first electron transport layer (ETL) 160, an n-type charge generation layer (n-CGL) 185, a p-type charge generation layer (p-GCL) 135 , a second hole transport layer (HTL) 141, a second electron blocking layer (EBL) 146, a second emission layer (EML) 151, an optional second hole blocking layer (HBL) 156, a second electron transport layer (ETL) 161, an electron injection layer (EIL) 181 and a cathode layer 190. The HIL may comprise a Ce(IV) metal complex or a Ce(IV) metal complex of formula (I).

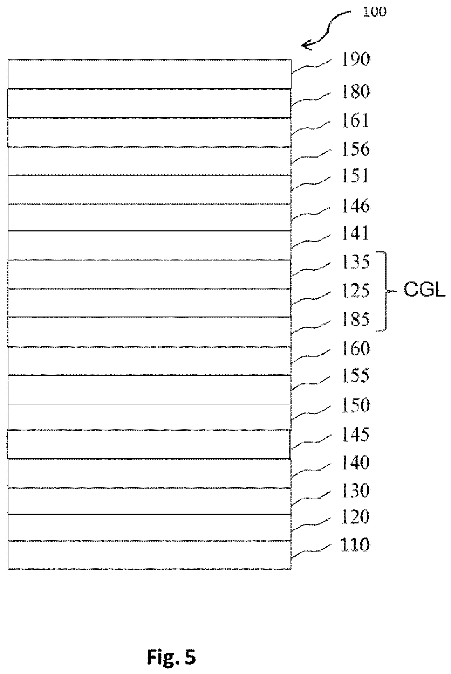

Fig. 5 is a schematic sectional view of a stacked OLED 100, according to another exemplary embodiment of the present invention. Fig. 5 differs from Fig. 4 in that the OLED 100 of Fig. 5 further comprises a charge generation connecting layer.

Referring to Fig. 5 the OLED 100 includes a substrate 110, an anode layer 120, a hole injection layer (HIL) 130, a first hole transport layer (HTL) 140, a first electron blocking layer (EBL) 145, a first emission layer (EML) 150, an optional first hole blocking layer (HBL) 155, a first electron transport layer (ETL) 160, an n-type charge generation layer (n-CGL) 185, a charge generation connecting layer 125 (c-CGL), a p-type charge generation layer (p-GCL) 135, a second hole transport layer (HTL) 141, a second electron blocking layer (EBL) 146, a second emission layer (EML) 151, an optional second hole blocking layer (HBL) 156, a second electron transport layer (ETL) 161, an electron injection layer (EIL) 181 and a cathode layer 190. The HIL may comprise a Ce(IV) metal complex or a Ce(IV) metal complex of formula (I).

While not shown in Fig. 1, Fig. 2, Fig. 3, Fig. 4 and Fig. 5, a capping and/or a sealing layer may further be formed on the cathode layer (190), in order to seal the organic electronic device 100. In addition, various other modifications may be applied thereto.

Hereinafter, the embodiments are illustrated in more detail with reference to examples. However, the present disclosure is not limited to the following examples.

### Detailed description

The invention is furthermore illustrated by the following examples which are illustrative only and non-binding.

The compound of formulae (IIIa) and (IIIb) and Ce(IV) metal complex, Ce(IV) metal complexes of formula (I) or formula (Ia) may be prepared by methods known in the art.

### Decomposition temperature T_{dec}

The decomposition temperature T_{dec} is measured by loading a sample of 9 to 11 mg into a Mettler Toledo 100 µL aluminum pan without lid under nitrogen in a Mettler Toledo TGA-DSC 1machine. The following heating program was used: 25°C isothermal for 3 min; 25°C to 600°C with 10 K/min.

The decomposition temperature was determined based on the onset of the decomposition in TGA.

The decomposition temperature indicates the temperature at which the compound decomposes. The higher the decomposition temperature the higher the thermal stability of a compound.

### Rate onset temperature

The rate onset temperature (T_{RO}) is determined by loading 100 mg compound into a VTE source. As VTE source a point source for organic materials may be used as supplied by Kurt J. Lesker Company (www.lesker.com) or CreaPhys GmbH (http://www.creaphys.com). The VTE source is heated at a constant rate of 15 K/min at a pressure of less than 10⁻⁵ mbar and the temperature inside the source measured with a thermocouple. Evaporation of the compound is detected with a QCM detector which detects deposition of the compound on the quartz crystal of the detector. The deposition rate on the quartz crystal is measured in Angstrom per second. To determine the rate onset temperature, the deposition rate is plotted against the VTE source temperature. The rate onset is the temperature at which noticeable deposition on the QCM detector occurs. For accurate results, the VTE source is heated and cooled three time and only results from the second and third run are used to determine the rate onset temperature.

To achieve good control over the evaporation rate of an organic compound, the rate onset temperature may be in the range of 100 to 300 °C. If the rate onset temperature is below 100 °C the evaporation may be too rapid and therefore difficult to control. If the rate onset temperature is above 300 °C the evaporation rate may be too low which may result in low tact time and decomposition of the organic compound in VTE source may occur due to prolonged exposure to elevated temperatures.

The rate onset temperature is an indirect measure of the volatility of a compound. The higher the rate onset temperature the lower is the volatility of a compound.

### HOMO

The HOMO energy is calculated with the program package TURBOMOLE V6.5 (TURBOMOLE GmbH, Litzenhardtstrasse 19, 76135 Karlsruhe, Germany) from the optimized geometry by applying the hybrid functional B3LYP with a Def-TZVP basis set in the gas phase and inclusion of dispersion forces as D3BJ. Geometry is optimized by applying the hybrid functional B3LYP with a Def-SVP basis set in the gas phase and inclusion of dispersion forces as D3BJ. If more than one conformation is viable, the conformation with the lowest total energy is selected.

If calculated by this method, the HOMO of N2,N2,N2',N2',N7,N7,N7',N7'-octakis(4-methoxyphenyl)-9,9'-spirobi[fluorene]-2,2',7,7'-tetraamine is -4.27 eV.

### LUMO

The LUMO energy is calculated as single point energy calculation with the program package TURBOMOLE V6.5 (TURBOMOLE GmbH, Litzenhardtstrasse 19, 76135 Karlsruhe, Germany) from the optimized geometry by applying the hybrid functional B3LYP with a Def-TZVP basis set in the gas phase and inclusion of dispersion forces as D3BJ. Geometry is optimized by applying the hybrid functional B3LYP with a Def-SVP basis set in the gas phase and inclusion of dispersion forces as D3BJ. If more than one conformation is viable, the conformation with the lowest total energy is selected.

### General procedure for fabrication of OLEDs comprising a n-CGL and a p-CGL

For bottom-emission OLEDs, see Table 3, a 15Ω /cm² glass substrate with 90 nm ITO (available from Corning Co.) was cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode.

Then 95 wt.-% organic hole transport compound F3 and 5 wt.-% 4,4',4"-((1E,1'E,1"E)-cyclopropane-1,2,3-triylidenetris(cyanomethanylylidene))tris(2,3,5,6-tetrafluorobenzonitrile) was vacuum co-deposited on the anode, to form a HIL having a thickness of 10 nm.

Then the same organic hole transport compound was vacuum deposited on the HIL, to form a first HTL having a thickness of 125 nm.

Then N,N-bis(4-(dibenzo[b,d]furan-4-yl)phenyl)-[1,1':4',1"-terphenyl]-4-amine was vacuum deposited on the first HTL, to form a first electron blocking layer (EBL1) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a first emission layer (EML1) with a thickness of 20 nm.

Then a first electron transport layer (ETL1) is formed on the first emission layer by depositing 2-(3'-(9,9-dimethyl-9H-fluoren-2-yl)-[1,1'-biphenyl]-3-yl)-4,6-diphenyl-1,3,5-triazine having a thickness of 25 nm.

Then, the n-type charge generation layer (n-CGL) having a thickness of 15 nm is formed on the ETL1 by co-depositing an organic electron transport compound ETM and a metal dopant. The composition of the n-CGL can be seen in Table 3.

Then, the p-type charge generation layer (p-CGL) having a thickness of 10 nm is formed on the n-CGL by co-depositing an organic hole transport compound HTM and a metal complex. The composition of the p-CGL can be seen in Table 3.

Then, a second hole transport layer (HTL2) having a thickness of 20 nm is formed on the p-CGL by depositing an organic hole transport compound as in the first hole transport layer. The composition of the second hole transport layer is the same as of the first hole transport layer.

Then N,N-bis(4-(dibenzo[b,d]furan-4-yl)phenyl)-[1,1':4',1"-terphenyl]-4-amine was vacuum deposited on the second HTL, to form a second electron blocking layer (EBL2) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a second emission layer (EML2) with a thickness of 20 nm.

Then a second electron transport layer (ETL2) is formed on the second emission layer by depositing 2-(3'-(9,9-dimethyl-9H-fluoren-2-yl)-[1,1'-biphenyl]-3-yl)-4,6-diphenyl-1,3,5-triazine having a thickness of 10 nm.

Then, the electron injection layer (EIL) having a thickness of 25 nm is formed on the second electron transport layer by co-depositing 99 vol.-% 3-Phenyl-3H-benzo[b]dinaphtho[2,1-d:1",2"-f]phosphepine-3-oxide and 1 vol.-% Yb.

Then Al is vacuum deposited on the electron injection layer at a rate of 0.01 to 1 Å/s at 10⁻⁷ mbar to form a cathode layer with a thickness of 100 nm.

The OLED stack is protected from ambient conditions by encapsulation of the device with a glass slide. Thereby, a cavity is formed, which includes a getter material for further protection.

### General procedure for fabrication of OLEDs comprising a n-CGL, a p-CGL and a c-CGL

For bottom-emission OLEDs, see Table 4, a 15Ω /cm² glass substrate with 90 nm ITO (available from Corning Co.) was cut to a size of 50 mm x 50 mm x 0.7 mm, ultrasonically washed with isopropyl alcohol for 5 minutes and then with pure water for 5 minutes, and washed again with UV ozone for 30 minutes, to prepare the anode.

Then 95 wt.-% organic hole transport compound F3 and 5 wt.-% 4,4',4"-((1E,1'E,1"E)-cyclopropane-1,2,3-triylidenetris(cyanomethanylylidene))tris(2,3,5,6-tetrafluorobenzonitrile) was vacuum co-deposited on the anode, to form a HIL having a thickness of 10 nm.

Then the same organic hole transport compound was vacuum deposited on the HIL, to form a first HTL having a thickness of 125 nm.

Then N,N-bis(4-(dibenzo[b,d]furan-4-yl)phenyl)-[1,1':4',1"-terphenyl]-4-amine was vacuum deposited on the first HTL, to form a first electron blocking layer (EBL1) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a first emission layer (EML1) with a thickness of 20 nm.

Then a first electron transport layer (ETL1) is formed on the first emission layer by depositing 2-(3'-(9,9-dimethyl-9H-fluoren-2-yl)-[1,1'-biphenyl]-3-yl)-4,6-diphenyl-1,3,5-triazine having a thickness of 25 nm.

Then, the n-type charge generation layer (n-CGL) having a thickness of 15 nm is formed on the ETL1 by co-depositing an organic electron transport compound ETM and a metal dopant. The composition of the n-CGL can be seen in Table 4.

Then, the charge generation connecting layer (c-CGL) having a thickness of 2 nm is formed on the n-CGL by co-depositing a conducting compound. The composition of the c-CGL can be seen in Table 4.

Then, the p-type charge generation layer (p-CGL) having a thickness of 10 nm is formed on the c-CGL by co-depositing an organic hole transport compound HTM and a metal complex. The composition of the p-CGL can be seen in Table 4.

Then, a second hole transport layer (HTL2) having a thickness of 20 nm is formed on the p-CGL by depositing an organic hole transport compound as in the first hole transport layer. The composition of the second hole transport layer is the same as of the first hole transport layer.

Then N,N-bis(4-(dibenzo[b,d]furan-4-yl)phenyl)-[1,1':4',1"-terphenyl]-4-amine was vacuum deposited on the second HTL, to form a second electron blocking layer (EBL2) having a thickness of 5 nm.

Then 97 vol.-% H09 (Sun Fine Chemicals, Korea) as EML host and 3 vol.-% BD200 (Sun Fine Chemicals, Korea) as fluorescent blue dopant were deposited on the EBL, to form a second emission layer (EML2) with a thickness of 20 nm.

Then a second electron transport layer (ETL2) is formed on the second emission layer by depositing 2-(3'-(9,9-dimethyl-9H-fluoren-2-yl)-[1,1'-biphenyl]-3-yl)-4,6-diphenyl-1,3,5-triazine having a thickness of 10 nm.

Then, the electron injection layer (EIL) having a thickness of 25 nm is formed on the second electron transport layer by co-depositing 99 vol.-% 3-Phenyl-3H-benzo[b]dinaphtho[2,1-d:1´,2´-f]phosphepine-3-oxide and 1 vol.-% Yb.

Then Al is vacuum deposited on the electron injection layer at a rate of 0.01 to 1 Å/s at 10⁻⁷ mbar to form a cathode layer with a thickness of 100 nm.

The OLED stack is protected from ambient conditions by encapsulation of the device with a glass slide. Thereby, a cavity is formed, which includes a getter material for further protection.

To assess the performance of the inventive examples compared to the prior art, the current efficiency is measured at 20 °C. The current-voltage characteristic is determined using a Keithley 2635 source measure unit, by sourcing a voltage in V and measuring the current in mA flowing through the device under test. The voltage applied to the device is varied in steps of 0.1V in the range between 0 V and 10 V. Likewise, the luminance-voltage characteristics and CIE coordinates are determined by measuring the luminance in cd/m² using an Instrument Systems CAS-140CT array spectrometer (calibrated by Deutsche Akkreditierungsstelle (DAkkS)) for each of the voltage values. The cd/A efficiency at 10 mA/cm² is determined by interpolating the luminance-voltage and current-voltage characteristics, respectively.

Lifetime LT of the device is measured at ambient conditions (20°C) and 30 mA/cm², using a Keithley 2400 source meter, and recorded in hours.

The brightness of the device is measured using a calibrated photo diode. The lifetime LT is defined as the time till the brightness of the device is reduced to 97 % of its initial value.

### Technical Effect of the invention

In Table 1 are shown the LUMO energy, decomposition temperatures T_{dec} and rate onset temperatures T_{RO} for Ce(IV) metal complexes of formula (I) and formula (Ia).

**Table 1: Ce(IV) metal complexes of formula (I) and formula (Ia)**

| Name | Chemical formula | LUMO (eV) | T_{dec} (°C) | T_{RO} (°C) |
|---|---|---|---|---|
| MC-1 | 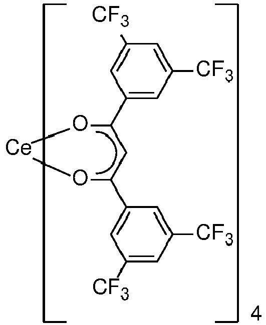 | -4.6 | > 440 | 270 |
| MC-2 | 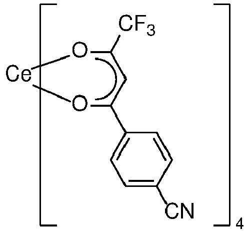 | -4.8 | n.d. ¹⁾ | n.d. |
| MC-3 | 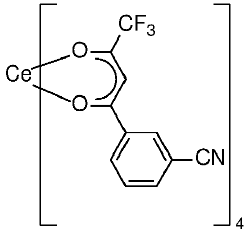 | -4.7 | n.d. | n.d. |
| MC-4 | 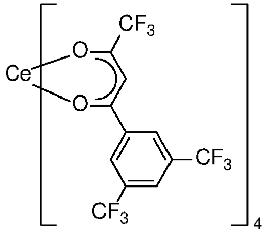 | -4.9 | > 270 | 122 |
| MC-5 | 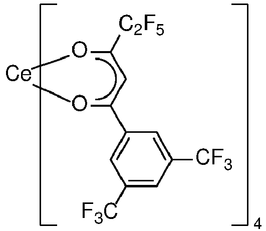 | -4.9 | n.d. | 120 |
| MC-6 | 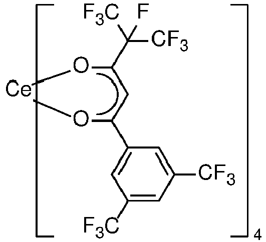 | -4.9 | n.d | n.d |
| MC-7 | 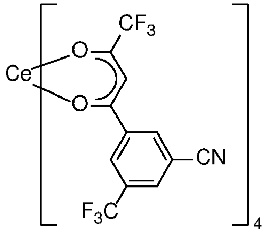 | -5.1 | n.d. | n.d. |
| MC-8 | 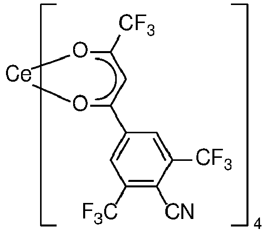 | -5.5 | n.d. | n.d. |
| MC-9 | 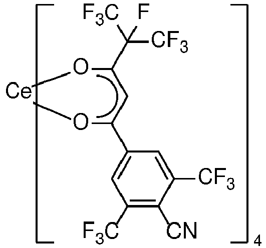 | -5.5 | n.d | n.d |
| MC-10 | 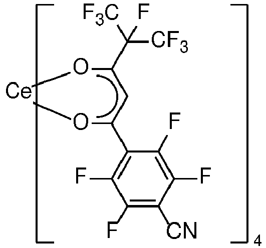 | -5.2 | n.d. | n.d. |
| MC-11 | 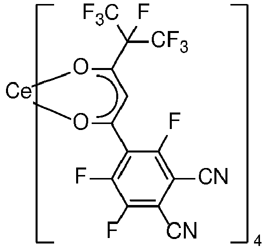 | -5.5 | n.d | n.d |
| MC-12 | 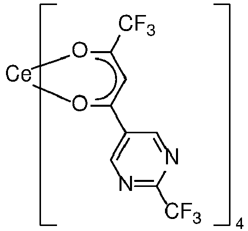 | -5.3 | n.d. | n.d. |
| MC-13 | 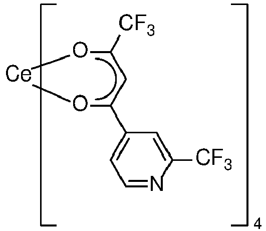 | -5.0 | n.d. | n.d. |
| MC-14 | 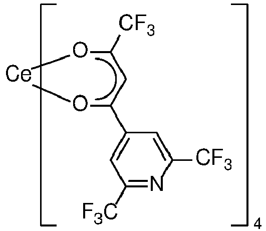 | -5.4 | n.d | n.d |
| MC-16 | 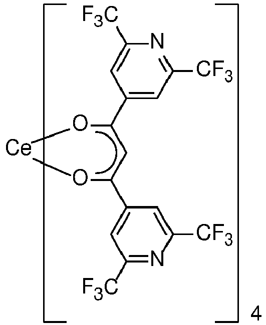 | -5.4 | n.d. | n.d. |

| | | | | |
|---|---|---|---|---|
| ¹⁾ n.d. = not determined | | | | |

As can be seen in Table 1, the Ce(IV) metal complexes of formula (I) or formula (Ia) have LUMO energies, decomposition temperatures and/or rate onset temperatures suitable for mass production of organic electronic devices.

In particular, the LUMO of Ce(IV) metal complexes of formula (I) or formula (Ia) are in the range suitable for doping of organic hole transport compounds of formula (IIIa) or (IIIb).

### Organic hole transport compound

In Table 2 are shown the HOMO energies and rate onset temperatures T_{RO} for hole transport compounds of formula (IIIa) or (IIIb). HOMO energies were calculated using TURBOMOLE V6.5 (TURBOMOLE GmbH, Litzenhardtstrasse 19, 76135 Karlsruhe, Germany) by applying the hybrid functional B3LYP with a Def-TZVP basis set in the gas phase with inclusion of dispersion as D3BJ.

**Table 2: Organic hole transport compounds of formula (IIIa) or (IIIb)**

| Name | Chemical formula | HOMO (eV) | T_{RO} (°C) |
|---|---|---|---|
| Biphenyl-4-yl(9,9-dipheny l-9H-fluoren-2-yl)-[4-(9-phenyl-9H-carbazol-3-yl)phenyl]-amine | 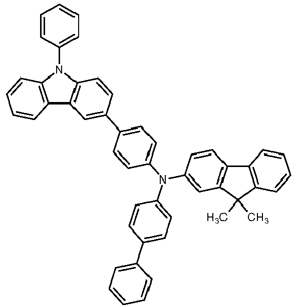 | -4.89 | 265 |
| F3 | | | |
| N,N'-Bis(naphthalen-1-yl)-N,N'-bis(phenyl)-benzidine | 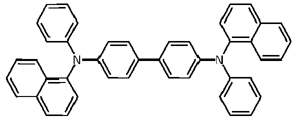 | -4.90 | 272 |
| F1 | | | |
| N4,N4"-di(naphthalen-1-yl)-N4,N4"-diphenyl-[1,1':4',1"-terphenyl]-4,4"-diamine | 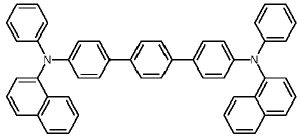 | -4.99 | 260 |
| F2 | | | |
| N-(9,9-dimethyl-9H-fluoren-2-yl)-N-(9,9-diphenyl-9H-fluoren-2-yl)dibenzo[b,d]fura n-1-amine | 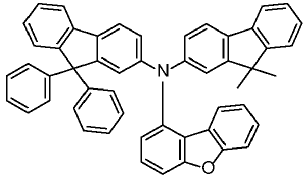 | -5.03 | 219 |
| F4 | | | |
| 9,9-dimethyl-N,N-bis(4-(naphthalen-1-yl)phenyl)-9H-fluoren-2-amine | 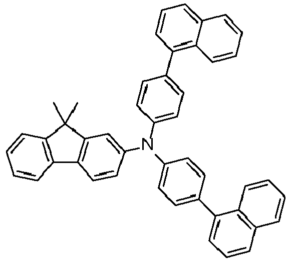 | -5.02 | 232 |
| F8 | | | |
| N-([1,1'-biphenyl]-4-yl)-N-(2-(9,9-dipheny l-9H-fluoren-4-yl)phenyl)-9,9-dimethyl-9H-fluoren-2-amine | 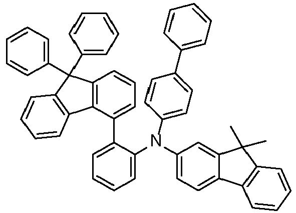 | -5.03 | 218 |
| F9 | | | |
| N-(9,9-diphenyl-9H-fluoren-2-yl)-N,9-diphenyl-9H-carbazol-2-amine | 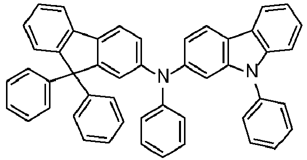 | -4.95 | 216 |
| F18 | | | |

As can be seen in Table 2, the organic hole transport compounds of formula (IIIa) or (IIIb) have rate onset temperatures suitable for mass production of organic electronic devices.

In Table 3 are shown data for organic electronic devices comprising a p-type charge generation layer comprising a Ce(IV) metal complex of formula (I) or formula (Ia) or comparative compound CC1, and an organic hole transport compound HTM; and a n-type charge generation layer comprising an organic electron transport compound ETM and a metal dopant.

In comparative example 1-1 and examples 1-1 and 1-2, the n-type charge generation layer comprises phosphine oxide compound ETM-1 as organic electron transport compound

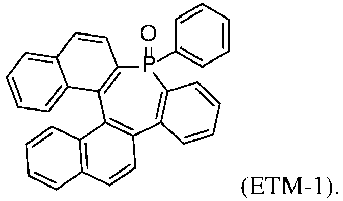

In comparative example 1-1, the p-type charge generation layer comprises comparative compound CC-1 as metal complex

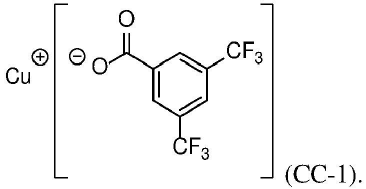

The operating voltage is 8.57 V, the cd/A efficiency is 3.08 cd/A and the lifetime is 12 hours.

In examples 1-1 and 1-2, the p-type charge generation layer comprises a Ce(IV) metal complex according to invention. As can be seen in Table 3, in examples 1-1 and 1-2, the operating voltage is reduced and the cd/A efficiency and/or lifetime are improved.

In examples 2-1 and 2-2, the OLED further comprises a connecting layer (c-CGL) between the n-CGL and the p-CGL. In examples 2-1 and 2-2, the connecting layer consists of ZnPc. As can be seen in Table 4, the operating voltage is reduced compared to comparative example 1-1. The cd/A efficiency and lifetime are improved further compared to Examples 1-1 and 1-2.

In summary, an improvement in operating voltage, cd/A efficiency and/or lifetime has been obtained.

A lower operating voltage and improved cd/A efficiency may be important for the battery life of organic electronic devices, in particular mobile devices.

An improvement in lifetime may be important for the long-term stability of organic electronic devices.

**Table 3: Performance of an organic electroluminescent device comprising a charge generation layer (CGL)**

| | n-type CGL | | | | | p-type CGL | | | | | | | |
|---|---|---|---|---|---|---|---|---|---|---|---|---|---|
| | ET M | Concentrat ion ETM (vol.-%) | Meta 1 dopa nt | Concentrat ion metal dopant (vol. %) | Thickn ess (nm) | HT M | Concentrat ion HTM (wt.-%) | Metal compl ex | Concentrat ion metal complex (wt.-%) | Thickn ess (nm) | Operati ng voltage at 10 mA/cm ² (V) | cd/A efficien cy at 10 mA/cm ² (cd/A) | LT97 at 30 mA/c m² (h) |
| Comparat ive example 1-1 | ET M-1 | 99 | Yb | 1 | 15 | F3 | 80 | CC-1 | 20 | 10 | 8.6 | 3.1 | 12 |
| Example 1-1 | ET M-1 | 99 | Yb | 1 | 15 | F3 | 80 | MC-4 | 20 | 10 | 7.5 | 12 | 74 |
| Example 1-2 | ET M-1 | 99 | Yb | 1 | 15 | F3 | 80 | MC-1 | 20 | 10 | 8.1 | 11.9 | 79 |

**Table 4: Performance of an organic electroluminescent device comprising a charge generation layer (CGL)**

| | n-type CGL | | | | | c-CGL | | p-type CGL | | | | | | | |
|---|---|---|---|---|---|---|---|---|---|---|---|---|---|---|---|
| | ET M | Concentr ation ETM (vol.-%) | Met al dop ant | Concentr ation metal dopant (vol. %) | Thick ness (nm) | Conduc ting compo und | Thick ness (nm) | HT M | Concentr ation HTM (wt.-%) | Metal comp lex | Concentr ation metal complex (wt.-%) | Thick ness (nm) | Operat ing voltag e at 10 mA/c m² (V) | cd/A efficie ncy at 10 mA/c m² (cd/A) | LT97 at 30 mA/c m² (h) |
| Exam ple 2-1 | ET M-1 | 99 | Yb | 1 | 15 | ZnPc | 2 | F3 | 90 | MC-4 | 10 | 10 | 7.7 | 12.8 | 90 |
| Exam ple 2-2 | ET M-1 | 99 | Yb | 1 | 15 | ZnPc | 2 | F3 | 80 | MC-4 | 20 | 10 | 7.3 | 12.75 | 87 |

The particular combinations of elements and features in the above detailed embodiments are exemplary only; the interchanging and substitution of these teachings with other teachings in this and the patents/applications incorporated by reference are also expressly contemplated. As those skilled in the art will recognize, variations, modifications, and other implementations of what is described herein can occur to those of ordinary skill in the art without departing from the spirit and the scope of the invention as claimed. Accordingly, the foregoing description is by way of example only and is not intended as limiting. In the claims, the word "comprising" does not exclude other elements or steps, and the indefinite article "a" or "an" does not exclude a plurality. The mere fact that certain measures are recited in mutually different dependent claims does not indicate that a combination of these measured cannot be used to advantage. The invention's scope is defined in the following claims and the equivalents thereto. Furthermore, reference signs used in the description and claims do not limit the scope of the invention as claimed.

## Claims

1. An organic electronic device comprising an anode layer, a cathode layer and a charge generation layer, wherein the charge generation layer comprises a p-type charge generation layer and a n-type charge generation layer,
wherein
- the n-type charge generation layer comprising an organic electron transport compound and a metal dopant, wherein the metal dopant is selected from a metal with an electronegativity of ≤ 1.4 eV by Pauling scale;
- the p-type charge generation layer comprising an organic hole transport compound and a Ce(IV) metal complex, wherein
the Ce(IV) metal complex comprises at least one anionic ligand L, wherein the anionic ligand comprising per ligand L at least 16 covalently bound atoms;
wherein the n-type charge generation layer is arranged closer to the anode layer and the p-type charge generation layer is arranged closer to the cathode layer.

2. An organic electronic device comprising an anode layer, a cathode layer and a charge generation layer, wherein the charge generation layer comprises a p-type charge generation layer and a n-type charge generation layer,
wherein
the n-type charge generation layer comprises an organic electron transport compound and a metal dopant; and
the p-type charge generation layer comprises:
- an organic hole transport compound, and
- a Ce(IV) metal complex, wherein the Ce(IV) metal complex has the formula (I):
Ce^{4⊕}(L^{⊖})₄(AL)ⁿ _{(I),}
wherein
L is an anionic ligand comprising at least 16 covalently bound atoms, wherein at least two atoms are selected from carbon atoms;
AL is an ancillary ligand which coordinates to the metal M;
n is an integer selected from 0 to 2;
wherein the n-type charge generation layer is arranged closer to the anode layer and the p-type charge generation layer is arranged closer to the cathode layer.

3. The organic electronic device according to claim 1 or 2, wherein the Ce(IV) metal complex has the formula (Ia): 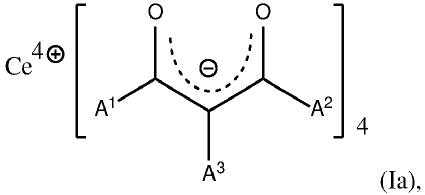 wherein
A¹ and A² are independently selected from substituted or unsubstituted C₁ to C₁₂ alkyl, substituted or unsubstituted C₆ to C₁₂ aryl, substituted or unsubstituted C₃ to C₁₂ heteroaryl, preferably substituted C₁ to C₁₂ alkyl, substituted C₆ to C₁₂ aryl, substituted C₃ to C₁₂ heteroaryl;
A³ is selected from H or D, preferably H;
wherein
the substituents of A¹ and A² are independently selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR¹, COOR¹, halogen, F or CN,
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C6 cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

4. The organic electronic device according to any of claims 2 or 3, wherein
- the substituents on A¹ and A² are independently selected from halogen, preferably F, C₁ to C₃ perhalogenated, perfluorinated alkyl or perfluorinated alkoxy, preferably perfluorinated C₁ to C₃ alkyl or perfluorinated C₁ to C₃ alkoxy, or -(O)₁-CₘH₂ₘ-CₙHaloₙ₂ₙ₊₁ with l= 0 or 1, preferably 0, m = 1 or 2, preferably 1 and n = 1 to 3, preferably n =1 or 2 and Halo = halogen, preferably F; and/or
- at least one of A¹ and A² is substituted alkyl and the substituents of the alkyl are fluorine with the number n_{F} (of fluorine substituents) and n_{H} (of hydrogens) follow the equation: n_{F} > n_{H} + 2; and/or
- at least one of A¹ and A² is selected from perfluorinated C₁ to C₆ alkyl, phenyl substituted with F or CF₃; and/or
- at least one of A¹ and A² is selected from perfluorinated alkyl or aryl.

5. The organic electronic device according to any of claims 2 to 4, wherein at least one of A¹ and A² comprises a substituent, wherein at least one of the substituents of A¹ and A² are independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; preferably at least one of A¹ and A² comprises at least two substituents, wherein the substituents on A¹ and A² are independently selected from C₃ to C₉ heteroaryl, C₁ to C₆ alkoxy, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkoxy, COR¹, COOR¹, halogen, F or CN; and further preferred A¹ and A² comprise at least one substituent independently selected from halogen, F, CF₃, C₂F₅, C₃F₇, C₄F₉, OCF₃, OC₂F₅, or CN; and in addition preferred A¹ and A² may comprise at least two substituents independently selected from halogen, F, CF₃, C₂F₅, C₃F₇, C₄F₉, OCF₃, OC₂F₅ or CN;
wherein R¹ is selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

6. The organic electronic device according to any of claims 2 to 5, wherein the sum of A¹ and A² comprise ≥ 3 carbon atoms and ≤ 30 carbon atoms.

7. The organic electronic device according to any of the preceding claims 1 to 6, wherein the organic hole transport compound is selected from the group comprising of at least one arylamine compound, diarylamine compound, triarylamine compound, a compound of formula (IIIa) or a compound of formula (IIIb): 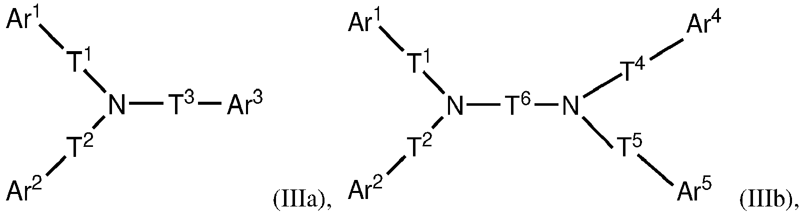 wherein:
T¹, T², T³, T⁴ and T⁵ are independently selected from a single bond, phenylene, biphenylene, terphenylene or naphthenylene, preferably a single bond or phenylene;
T⁶ is phenylene, biphenylene, terphenylene or naphthenylene;
Ar¹, Ar², Ar³, Ar⁴ and Ar⁵ are independently selected from substituted or unsubstituted C₆ to C₂₀ aryl, or substituted or unsubstituted C₃ to C₂₀ heteroarylene, substituted or unsubstituted biphenylene, substituted or unsubstituted fluorene, substituted 9-fluorene, substituted 9,9-fluorene, substituted or unsubstituted naphthalene, substituted or unsubstituted anthracene, substituted or unsubstituted phenanthrene, substituted or unsubstituted pyrene, substituted or unsubstituted perylene, substituted or unsubstituted triphenylene, substituted or unsubstituted tetracene, substituted or unsubstituted tetraphene, substituted or unsubstituted dibenzofurane, substituted or unsubstituted dibenzothiophene, substituted or unsubstituted xanthene, substituted or unsubstituted carbazole, substituted 9-phenylcarbazole, substituted or unsubstituted azepine, substituted or unsubstituted dibenzo[b,f]azepine, substituted or unsubstituted 9,9'-spirobi[fluorene], substituted or unsubstituted spiro[fluorene-9,9'-xanthene], or a substituted or unsubstituted aromatic fused ring system comprising at least three substituted
or unsubstituted aromatic rings selected from the group comprising substituted or unsubstituted non-hetero, substituted or unsubstituted hetero 5-member rings, substituted or unsubstituted 6-member rings and/or substituted or unsubstituted 7-member rings, substituted or unsubstituted fluorene, or a fused ring system comprising 2 to 6 substituted or unsubstituted 5- to 7-member rings and the rings are selected from the group comprising (i) unsaturated 5- to 7-member ring of a heterocycle, (ii) 5- to 6-member of an aromatic heterocycle, (iii) unsaturated 5- to 7-member ring of a non-heterocycle, (iv) 6-member ring of an aromatic non-heterocycle;
wherein
the substituents of Ar¹, Ar², Ar³, Ar⁴ and Ar⁵ are selected the same or different from the group comprising H, D, F, C(-O)R², CN, Si(R²)₃, P(-O)(R²)₂, OR², S(-O)R², S(-O)₂R², substituted or unsubstituted straight-chain alkyl having 1 to 20 carbon atoms, substituted or unsubstituted branched alkyl having 1 to 20 carbon atoms, substituted or unsubstituted cyclic alkyl having 3 to 20 carbon atoms, substituted or unsubstituted alkenyl or alkynyl groups having 2 to 20 carbon atoms, substituted or unsubstituted alkoxy groups having 1 to 20 carbon atoms, substituted or unsubstituted aromatic ring systems having 6 to 40 aromatic ring atoms, and substituted or unsubstituted heteroaromatic ring systems having 5 to 40 aromatic ring atoms, unsubstituted C₆ to C₁₈ aryl, unsubstituted C₃ to C₁₈ heteroaryl, a fused ring system comprising 2 to 6 unsubstituted 5- to 7-member rings and the rings are selected from the group comprising unsaturated 5- to 7-member ring of a heterocycle, 5- to 6-member of an aromatic heterocycle, unsaturated 5- to 7-member ring of a non-heterocycle, and 6-member ring of an aromatic non-heterocycle,
wherein R² may be selected from H, D, straight-chain alkyl having 1 to 6 carbon atoms, branched alkyl having 1 to 6 carbon atoms, cyclic alkyl having 3 to 6 carbon atoms, alkenyl or alkynyl groups having 2 to 6 carbon atoms, C₆ to C₁₈ aryl or C₃ to C₁₈ heteroaryl.

8. The organic electronic device according to any of the preceding claims 1 to 7, wherein the metal dopant is selected from a metal with an electronegativity of ≤ 1.35 eV by Pauling scale; preferably the metal dopant is a metal selected from the group comprising Li, Na, K, Rb, Cs, Mg, Ca, Sr, Ba, Sm, Eu or Yb, more preferred the metal dopant is a metal selected from the group comprising Li, Na, K, Cs, Mg, Ca, Ba, Sm, Eu or Yb, even more preferred the metal dopant is a metal selected from the group comprising Li, Mg or Yb.

9. The organic electronic device according to any of the preceding claims 1 to 8, wherein the AL is selected from the group comprising H₂O, C₂ to C₄₀ mono- or multi-dentate ethers and C₂ to C₄₀ thioethers, C₂ to C₄₀ amines, C₂ to C₄₀ phosphine, C₂ to C₂₀ alkyl nitrile or C₂ to C₄₀ aryl nitrile, or a compound according to Formula (II); 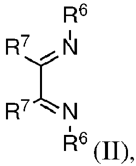 wherein
R⁶ and R⁷ are independently selected from C₁ to C₂₀ alkyl, C₁ to C₂₀ heteroalkyl, C₆ to C₂₀ aryl, heteroaryl with 5 to 20 ring-forming atoms, halogenated or perhalogenated C₁ to C₂₀ alkyl, halogenated or perhalogenated C₁ to C₂₀ heteroalkyl, halogenated or perhalogenated C₆ to C₂₀ aryl, halogenated or perhalogenated heteroaryl with 5 to 20 ring-forming atoms, or at least one R⁶ and R⁷ are bridged and form a 5 to 20 member ring, or the two R⁶ and/or the two R⁷ are bridged and form a 5 to 40 member ring or form a 5 to 40 member ring comprising an unsubstituted or C₁ to C₁₂ substituted phenanthroline.

10. The organic electronic device according to any of claims 1 to 9, wherein
the ligand L of formula I comprising per ligand L at least 14 covalently bound atoms; or the ligand L of formula I comprising:
- at least three carbon atoms, alternatively at least four carbon atoms, and/or
- at least two oxygen atoms or one oxygen and one nitrogen atom, two to four oxygen atoms, two to four oxygen atoms and zero to two nitrogen atoms, and/or
- at least one or more groups selected from halogen, F, CN, substituted or unsubstituted C₁ to C₆ alkyl, substituted or unsubstituted C₁ to C₆ alkoxy, alternatively two or more groups selected from halogen, F, CN, substituted or unsubstituted C₁ to C₆ alkyl, substituted or unsubstituted C₁ to C₆ alkoxy, at least one or more groups selected from halogen, F, CN, substituted C₁ to C₆ alkyl, substituted C₁ to C₆ alkoxy, alternatively two or more groups selected from halogen, F, CN, perfluorinated C₁ to C₆ alkyl, perfluorinated C₁ to C₆ alkoxy, one or more groups selected from substituted or unsubstituted C₁ to C₆ alkyl, substituted or unsubstituted C₆ to C₁₂ aryl, and/or substituted or unsubstituted C₃ to C₁₂ heteroaryl,
wherein the substituents are selected from D, C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy, COR³, COOR³, halogen, F or CN;
wherein R³ may be selected from C₆ aryl, C₃ to C₉ heteroaryl, C₁ to C₆ alkyl, C₁ to C₆ alkoxy, C₃ to C₆ branched alkyl, C₃ to C₆ cyclic alkyl, C₃ to C₆ branched alkoxy, C₃ to C₆ cyclic alkoxy, partially or perfluorinated C₁ to C₁₆ alkyl, partially or perfluorinated C₁ to C₁₆ alkoxy, partially or perdeuterated C₁ to C₆ alkyl, partially or perdeuterated C₁ to C₆ alkoxy.

11. The organic electronic device according to any of claims 1 to 10, wherein the ligand L of formula I are selected from G1 to G60:

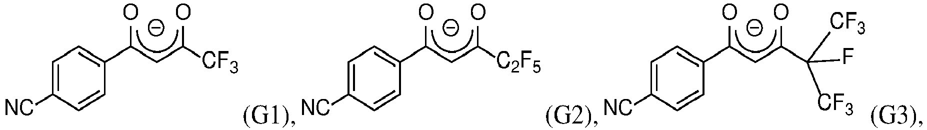

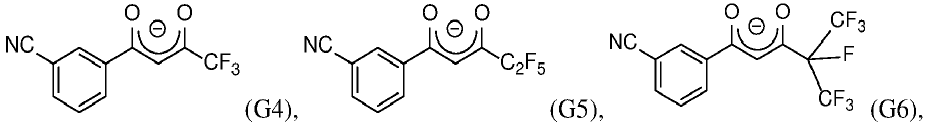

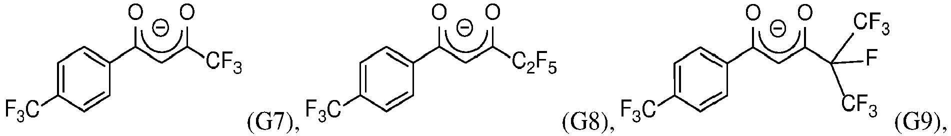

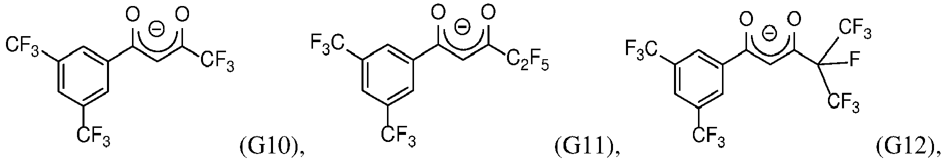

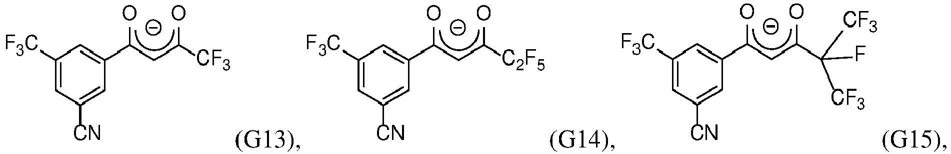

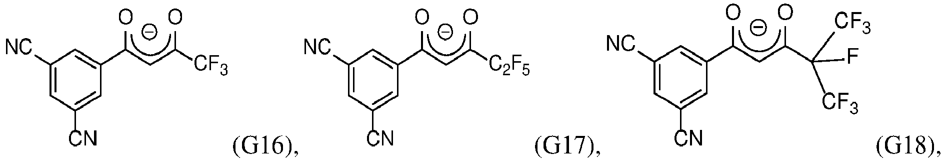

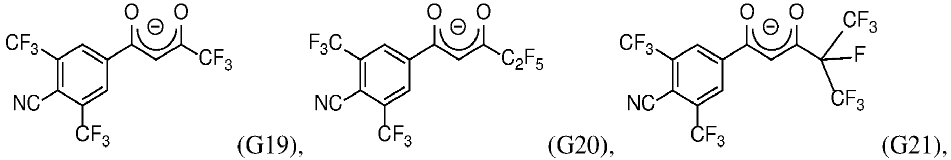

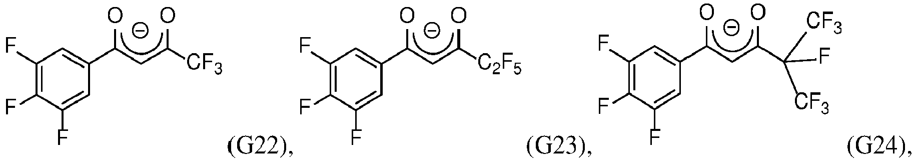

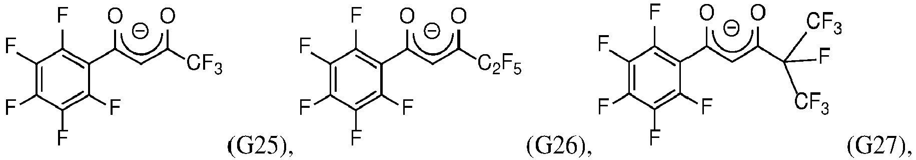

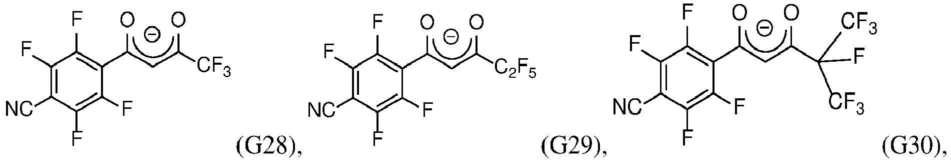

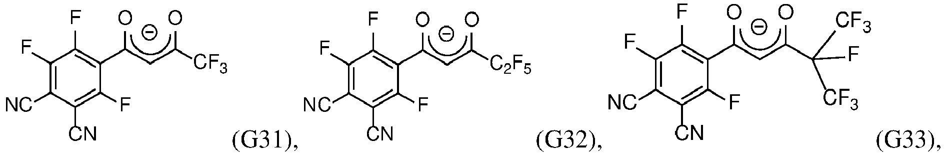

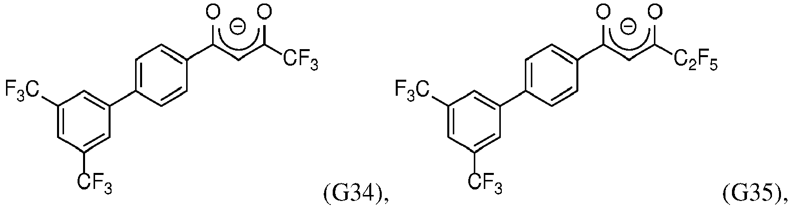

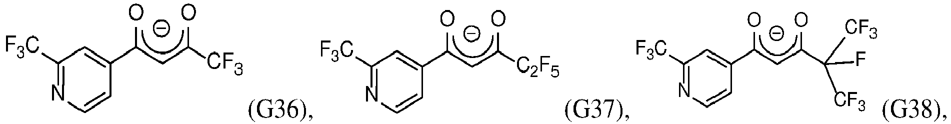

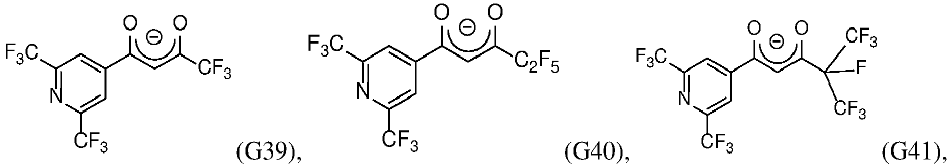

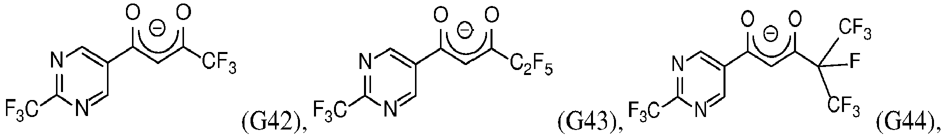

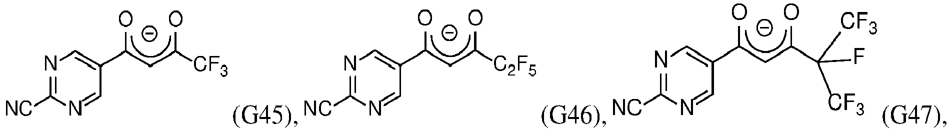

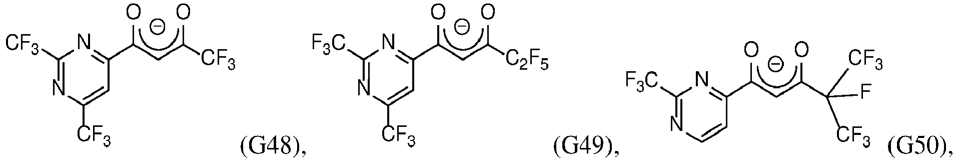

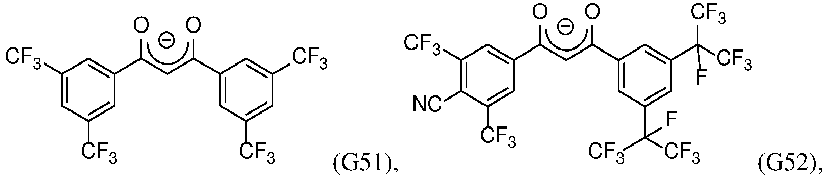

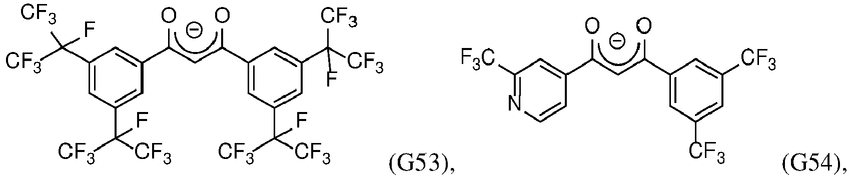

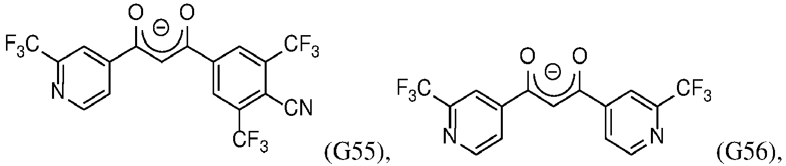

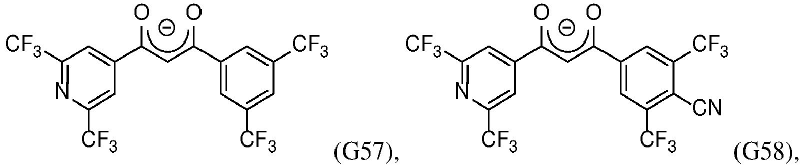

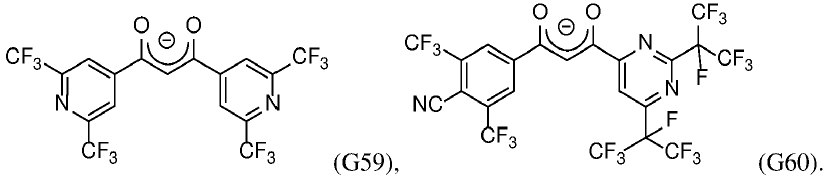

12. The organic electronic device according to any of claims 2 to 11, wherein A¹ and/or A² are selected from the following Formulas D1 to D68: 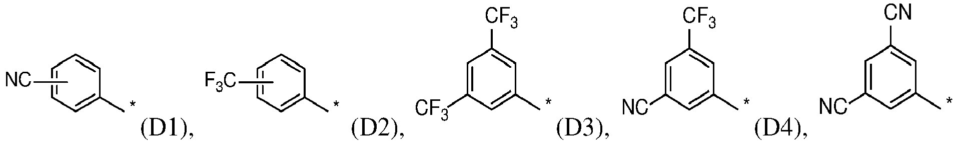 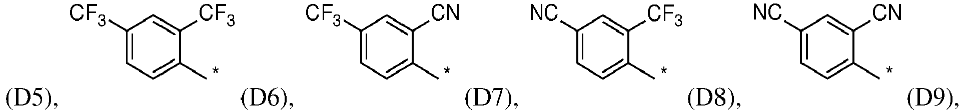 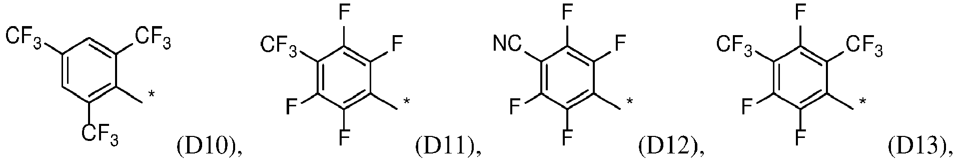 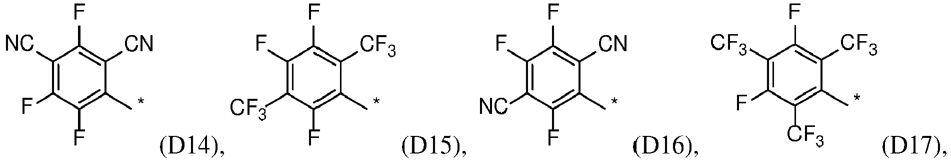 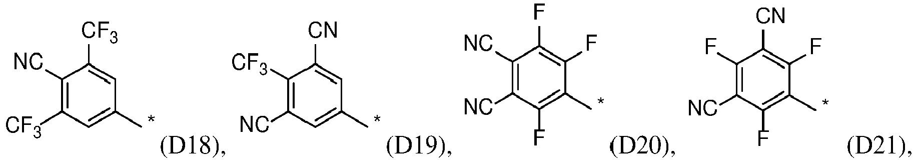 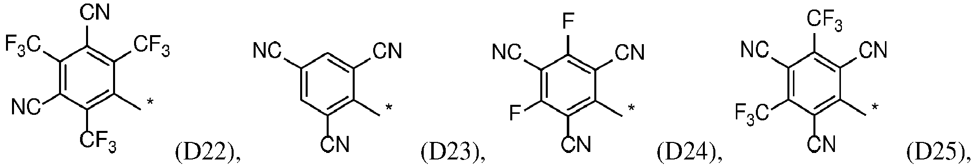 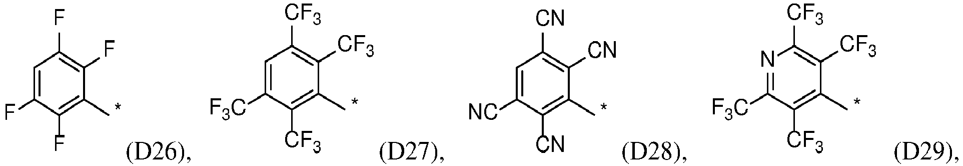 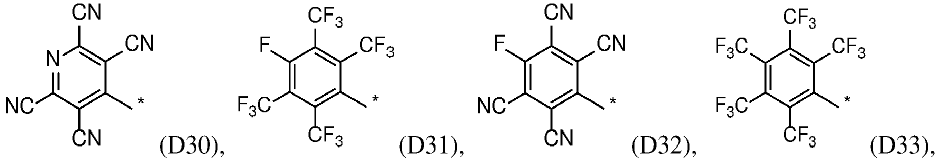 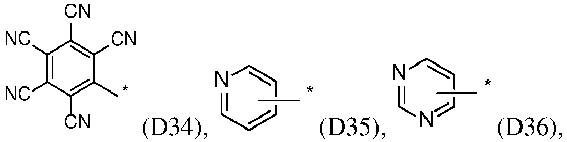 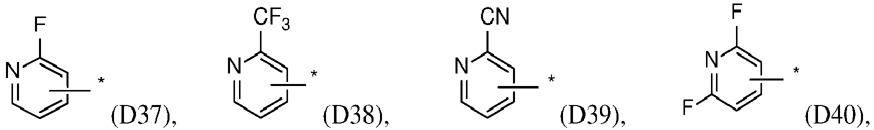 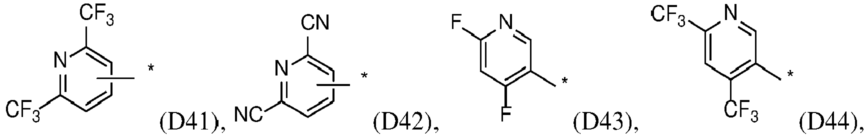 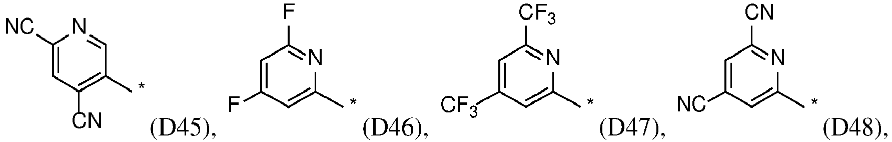 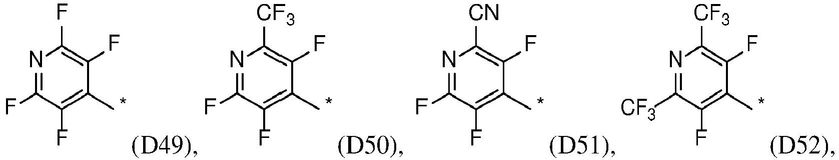 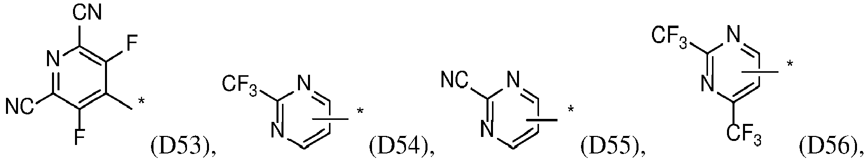 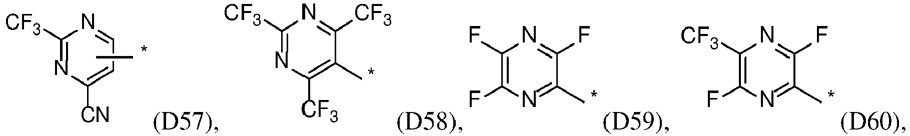 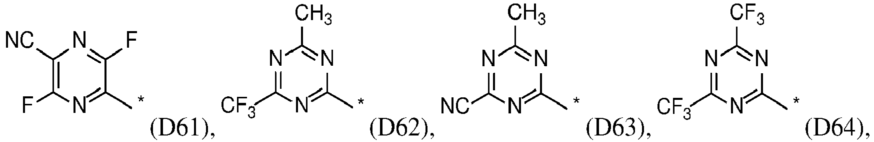 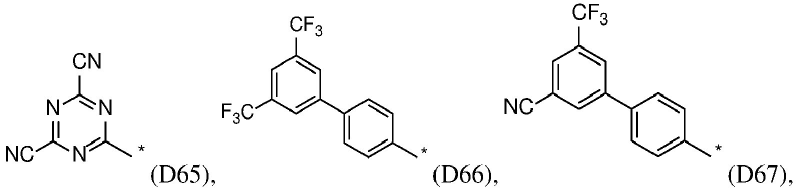 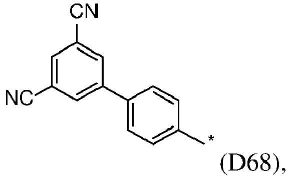 wherein the "^{∗}" denotes the binding position.

13. The organic electronic device according to any of claims 1 to 12, wherein the Ce(IV) metal complex of formula (I) are selected from Ce(IV) metal complexes E1 to E17:

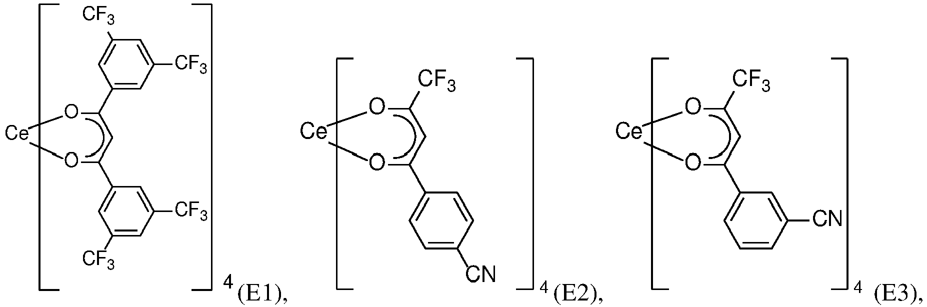

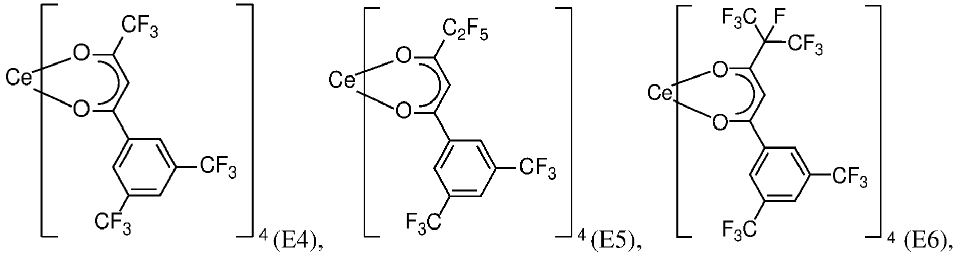

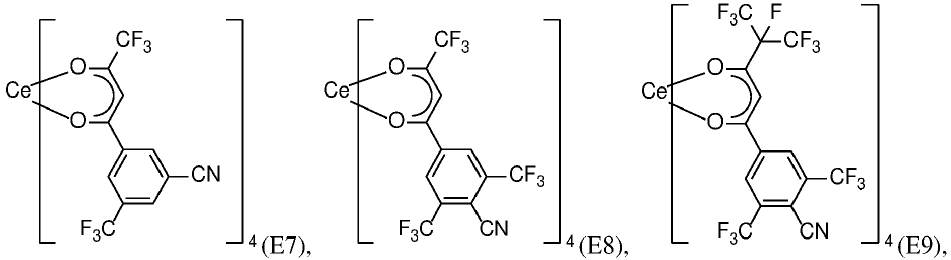

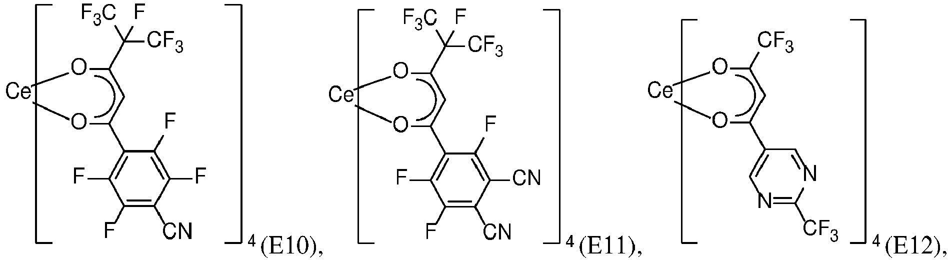

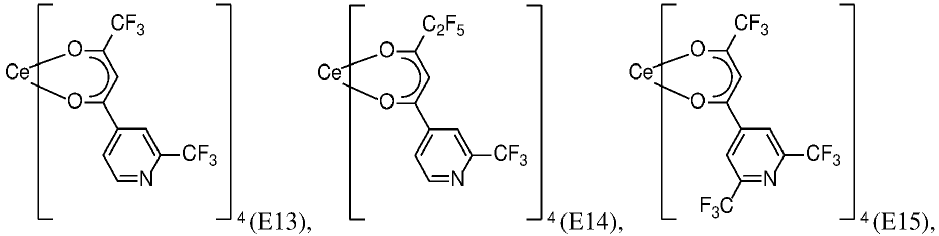

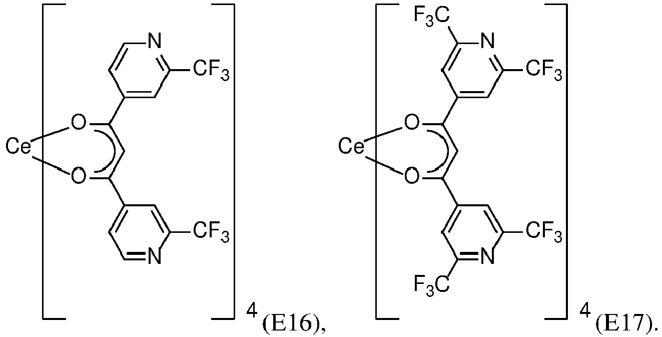

14. The organic electronic device according to any of the preceding claims 1 to 13, wherein the organic electronic device is a light emitting device or a display device.

15. A Ce(IV) metal complex of formula (Ia): 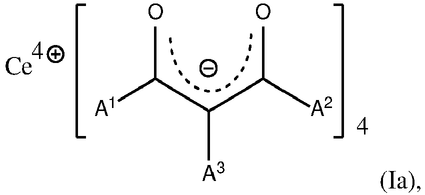 wherein
A¹ is selected from formulae D1 and D4: 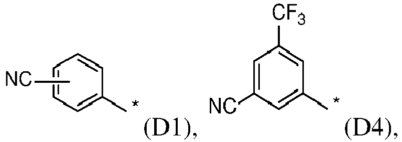
wherein the "^{∗}" denotes the binding position;
A² is selected from substituted or unsubstituted C₁ to C₁₂ alkyl, wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
A³ is selected from H or D, preferably H; or
wherein
A¹ is selected from formulae D39, D42 and D56: 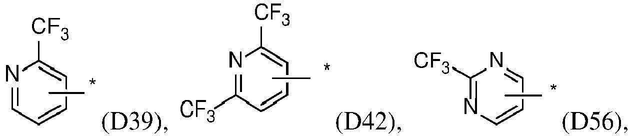
wherein the "^{∗}" denotes the binding position;
A² is selected from substituted or unsubstituted C₁ to C₁₂ alkyl, unsubstituted or substituted C6 to C₁₂ aryl, substituted or unsubstituted C₃ to C₁₂ heteroaryl; wherein the substituents of A² are independently selected from D, C₃ to C₆ branched alkyl, C₃ to C₆ cyclic alkyl, partially or perfluorinated C₁ to C₁₆ alkyl, partially or perdeuterated C₁ to C₆ alkyl, halogen, F or CN; and
A³ is selected from H or D, preferably H.
